# EUROPÄISCHE PATENTSCHRIFT

(11) **EP 2 411 032 B1**
(45) Veröffentlichungstag und Bekanntmachung des Hinweises auf die Patenterteilung: **09.07.2014**
(21) Anmeldenummer: 10719722.0
(22) Anmeldetag: 29.03.2010
(51) Int. Cl.: A61Q 19/00, A61Q 19/08, A61K 8/92, A61K 8/97, A61K 36/88, A61P 17/06, A61P 17/10, A61P 17/00

(54) **PFLANZEN-EXTRAKTE ZUR BEHANDLUNG VON HAUTERKRANKUNGEN**
PLANT EXTRACTS FOR THE TREATMENT OF SKIN DISEASES
EXTRAITS DE PLANTES POUR LE TRAITEMENT DE MALADIES DE LA PEAU

(30) Priorität: 27.03.2009 DE 102009014834
(43) Veröffentlichungstag der Anmeldung: 01.02.2012
(73) Patentinhaber: Evoria GmbH, 41460 Neuss (DE)
(72) Erfinder: SUSILO, Rudy, 14089 Berlin (DE)
(74) Vertreter: Arth, Hans-Lothar
(86) Internationale Anmeldenummer: PCT/DE2010/000359
(87) Internationale Veröffentlichungsnummer: WO 2010/108488

(56) Entgegenhaltungen:
- WO-A2-2007/048398
- DATABASE WPI Week 200381 Thomson Scientific, London, GB; AN 2003-869286 XP002589555 & JP 2003 267851 A (NOEVIR KK) 25. September 2003 (2003-09-25)
- DATABASE WPI Week 200416 Thomson Scientific, London, GB; AN 2004-162176 XP002589558 & JP 2004 043392 A (NOEVIR KK) 12. Februar 2004 (2004-02-12)
- DATABASE WPI Week 200718 Thomson Scientific, London, GB; AN 2007-181784 XP002589584 & JP 2007 031287 A (HOSODA SHOTEN KK) 8. Februar 2007 (2007-02-08)
- DATABASE WPI Week 200416 Thomson Scientific, London, GB; AN 2004-162177 XP002589585 & JP 2004 043393 A (NOEVIR KK) 12. Februar 2004 (2004-02-12)
- BOURKE R M: "Some little-known food crops from Papua New Guinea" SCIENCE IN NEW GUINEA,, Bd. 8, Nr. 3, 1. Januar 1981 (1981-01-01), Seiten 164-170, XP009135489
- DATABASE WPI Week 200559, Derwent Publications Ltd., London, GB; AN 2005-571974 & CN 1 605 356 A (UNIV BIOPHARMACEUTIAL RES & DEV CENT JINAN) 13 April 2005
- CHEN H ET AL: "A study of microemulsion systems for transdermal delivery of triptolide", JOURNAL OF CONTROLLED RELEASE, ELSEVIER, AMSTERDAM, NL, vol. 98, no. 3, 27 August 2004 (2004-08-27), pages 427-436, XP004527195, ISSN: 0168-3659, DOI: 10.1016/J.JCONREL.2004.06.001
- CHANCHAL D ET AL: "Novel approaches in herbal cosmetics", JOURNAL OF COSMETIC DERMATOLOGY, BLACKWELL SCIENCE, OXFORD, GB, vol. 7, no. 2, 1 June 2008 (2008-06-01), pages 89-95, XP009164387, ISSN: 1473-2130
- SARKER D K: "ENGINEERING OF NANOEMULSIONS FOR DRUG DELIVERY", CURRENT DRUG DELIVERY, BENTHAM SCIENCE PUBLISHERS, HILVERSUM, NL, vol. 2, no. 4, 1 October 2005 (2005-10-01) , pages 297-310, XP001246683, ISSN: 1567-2018, DOI: 10.2174/156720105774370267

## Beschreibung

Die Erfindung betrifft die Verwendung eines Extraktes oder von Extrakten, von lipophilen Extrakten als auch hydrophilen Extrakten aus der Frucht der Pflanze *Pandanus conoideus* zur Herstellung von topischen Formulierungen und insbesondere von Emulsionen zur Behandlung und Prophylaxe von Neurodermitis, Dermatitis, Psoriasis oder Akne.

Neurodermitis wird auch als atopisches Ekzem, endogenes Ekzem chronisch konstitutionelles Ekzem, Asthmaekzem, Prurigo Besnier oder atopische Dermatitis bezeichnet. Die Krankheit ist chronisch und nicht ansteckend. Ferner gilt sie als behandelbar jedoch zur Zeit noch immer als unheilbar. Die sich in roter, trockener, schuppender und manchmal mit nässender Ekzeme auf der Haut äußernde Krankheit wird meist von einem starken Juckreiz begleitet und in der Regel durch die topische Anwendung entzündungshemmender Stoffe behandelt.

Die Ursachen der Neurodermitis sind bislang nicht vollständig geklärt. Experten sehen im komplexen Krankheitsgeschehen und seinem sehr individuellen Verlauf ein Zusammenspiel aus genetischen Faktoren, psychischen Einflüssen, immunologischen Veränderungen und Umwelteinflüssen. Neuere Ergebnisse zeigen, dass die Barriere-Funktion der Haut bei atopischer Dermatitis entscheidend beeinträchtigt ist. Insbesondere durch Störungen der Lipid-Barriere-Funktion (Haut-Lipid-Barriere) verliert die Haut vermehrt Wasser. Das atopische Ekzem äußert sich daher insbesondere durch eine empfindliche und trockene Haut, die oft auch gerötet ist. Die geschwächte Barriere verursacht ein erleichtertes Eindringen von Allergenen und die erhöhte Entzündungsbereitschaft atopischer Haut. Die Haut ist anfällig für äußere Reize, die zu Juckreiz führen können.

Bisher sind noch keine speziellen verlässfichen Untersuchungen bekannt, mit der die Neurodermitis diagnostiziert werden kann. Der Arzt erstellt typischerweise eine umfassende Anamnese, in der die Krankheitsgeschichte und familiäre Umstände berücksichtigt werden. Die anschließende Körperliche Untersuchung unterscheidet weiterhin Diagnostische Kriterien erster und zweiter Ordnung, wobei die Diagnose Neurodermitis getroffen wird, sobald ein Patient mindestens drei Merkmale der ersten Ordnung und mindestens drei Merkmale der zweiten Ordnung aufweist.

Diagnostische Kriterien der ersten Ordnung umfassen unter anderem Ekzeme an Stellen, die für die Neurodermatitis besonders typisch sind, wie an den Kniekehlen, Ellenbeugen, Nacken, Hals und Gesicht, weiterhin starker Juckreiz, ein chronischer Verlauf der Krankheit sowie das Vorkommen von Erkrankungen in der Familie an Neurodermitis, Heuschnupfen oder Asthma.

Diagnostische Kriterien der zweiten Ordnung umfassen unter anderem Juckreiz beim Schwitzen, dunkle Augenringe, Blässe rund um den Mund, eine angeborene doppelte untere Lidfalte oder stärkere Zeichnung der Hautlinien, positive Allergietests, Ichthyosis, Xerosis oder Anfälligkeit gegenüber Hautinfektionen. Weiterhin zeigen Neurodermitis Patienten eine Neigung zur Verengung der Blutgefäße im Bereich der Haut, die sich dadurch ausdrückt, dass ein sogenannter weißer Dermographismus auftreten kann, bei dem z.B. nach Kratzen keine roten Striemen auf der Haut zurückbleiben sondern weiße.

Hierbei kann es sich nicht um eine abschließende Aufzählung handeln, da die Ursachen der Neurodermitis nicht vollständig geklärt sind und das sehr komplexe Krankheitsgeschehen einen sehr individuellen Verlauf hat. Die Diagnose Neurodermitis kann damit auch zutreffen, wenn weniger als drei Diagnostische Kriterien vorliegen, diese aber vom Arzt als so schwer eingestuft werden, dass eine Neurodermitis als sehr wahrscheinlich gilt.

*Pandanus conoideus* ist eine endemische Pflanzenart aus Irian Jaya, Indonesien. Die Früchte dieser Pflanze finden Anwendung bei den Einheimischen als Nahrungsmittel, Nahrungsmittelergänzung, Viehfutter, natürlicher Farbstoff und als Heilmittel gegen verschiedenste Erkrankungen wie Würmererkrankungen und Blindheit.

Die traditionelle Zubereitung erfolgt durch langes Kochen der roten äußeren Fruchtschicht und anschließendem Zerreiben des Fruchtfleisches, um einen kernfreien Brei zu erhalten. Der daraus gewonnene ölige Brei wird als tägliches Lebensmittel oder als Heilmittel eingesetzt.

Die *Pandanus conoideus* ist eine kürbisartige längliche Frucht, welche im Inneren ein kernloses weißes helles Fruchtfleisch besitzt und darum herum befindet sich das rote Fruchtfleisch, worin sich auch die Kerne der Frucht befinden. Außen wird die zylindrische im Durchschnitt 1 m lange, 7,5 kg schwere und mit einem Durchmesser von 12 cm recht große Frucht von einer roten Schale umgeben.

Aufgabe der vorliegenden Erfindung ist es, ein Mittel zur Anwendung, Prophylaxe und Behandlung von verschiedenen Hauterkrankungen wie beispielsweise Akne, Neurodermitis, Dermatitis, oder Psoriasis bereitzustellen.

Diese Aufgabe wird durch die technische Lehre der unabhängigen Patentansprüche gelöst. Vorteilhafte Ausgestaltungen der Erfindung sind in den abhängigen Patentansprüchen, der Beschreibung sowie den Beispielen angegeben.

Überraschenderweise wurde gefunden, dass die Extrakte der Spezies *Pandanus conoideus* als kosmetische sowie pharmazeutische Mittel zur Behandlung und Prophylaxe von insbesondere Neurodermitis, Dermatitis und Ekzemen sowie zur kosmetischen Anwendung bei trockener Haut, Stoffwechselstörungen der Haut, Akne, Psoriasis, geschädigter Haut und Alterungserscheinungen der Haut sowie zur Behandlung von Schleimhäuten im Bereich von Nase, Augen und Ohren erfolgreich eingesetzt werden können.

Es gibt unterschiedliche Formen und Farben der Früchte von *Pandanus conoideus,* wie beispielsweise längliche rote Früchte, kurze rote Früchte, braune und gelbe Früchte. Alle diese Früchte können für die erfindungsgemäßen Zwecke und Verwendungen eingesetzt werden.

Überraschenderweise wurde gefunden, dass die erfindungsgemäßen Pandanusextrakte sich exzellent zur Behandlung von Neurodermitis eignen und in einer klinischen Studie an 20 Patienten mit Neurodermitis wurde bereits nach 14 tägiger Anwendung eine Reduktion des durchschnittlichen Atopie-Score von 30,79 auf 18,04. Dies entspricht einer Verminderung um -41,40 % zum Ausgangswert. Bei 21 tätiger Anwendung ergab sich eine Reduktion des durchschnittlichen Atopie-Score von 30,79 auf 6,4. Dies entspricht einer Verminderung um -79,23 % zum Ausgangswert. Dieser Behandlungserfolg kann nochmals durch Verwendung spezieller Formulierungen wie beispielsweise Mikroemulsionen oder Nanoemulsionen gesteigert werden.

Die kosmetischen und pharmazeutischen Pandanus-Extrakt Präparate liegen besonders bevorzugt als Emulsion vor, wie z.B. einer Emulsion vom Typ Wasserin-Öl (W/O) oder vom Typ Öl-in-Wasser (O/W). Unter Emulsionen versteht man heterogene Gemische, die aus zwei nicht miteinander mischbaren Flüssigkeiten bestehen, wobei eine der beiden Flüssigkeiten feinst verteilt als kleine Tröpfchen in der anderen Flüssigkeit vorliegt.
Der Begriff "Emulsion" wie hierin verwendet bezeichnet einerseits das System von Öltröpfchen fein verteilt in einer Wasserphase oder Wassertröpfchen fein verteilt in einer Ölphase als auch andererseits die fertige kosmetische oder pharmazeutische Formulierung für den Endverbraucher.
Um das galenische System der Emulsion von der verbrauchsfertigen Formulierung in Form einer Emulsion zu trennen, wird hierin auch teilweise der Begriff Emulsionsformulierung für die verbrauchsfertigen Formulierung in Form einer Emulsion verwendet.
Erfindungsgemäß sind Emulsionsformulierungen, Salben, Pasten, Cremes oder Gele für die verbrauchsfertige Form bevorzugt. Diese Formen der Endprodukte, d.h. diese Formen für das verbrauchsfertige Produkt enthalten vorzugsweise das System der Emulsion und insbesondere bevorzugt Mikroemulsionen oder Nanoemulsionen. Dies bedeutet, dass vorzugsweise der Pandanusextrakt in einer Ölphase verteilt wird und die Ölphase in Form kleiner Tröpfchen in einer wässrigen Phase verteilt wird. Dieses galenische System einer Emulsion kann in der vorliegenden Form als verbrauchsfertige Emulsionsformulierung angewendet werden oder diese Emulsion kann Bestandteil einer Creme, Paste, Salbe oder eines Gels sein, wobei die Creme, Paste, Salbe oder das Gel dann die verbrauchsfertige Endformulierung darstellen. Besonders bevorzugt ist die Verwendung von Mikroemulsionen oder Nanoemulsionen als gebrauchsfertige Emulsionsformulierung oder als Bestandteil einer Creme, Paste, Salbe oder eines Gels. Nanoemulsionen sind im Vergleich zu Mikroemulsionen nochmals bevorzugt.

Als Mikroemulsionen werden Emulsionen bezeichnet, welche eine durchschnittliche Tröpfengröße der Öltropfen von 1 µm bis 50 µm aufweisen und als Nanoemulsionen werden Emulsionen bezeichnet, welche eine durchschnittliche Tröpfengröße der Öltropfen von 50 nm bis 800 nm aufweisen.

Die Ölphase der erfindungsgemäßen Präparate wird ausgewählt aus dem Pandanusextrakt alleine oder eine Mischung von Pandanusextrakt mit anderen natürlichen und/oder synthetischen Ölen. Die anderen Öle werden ausgewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Diese Esteröle werden vorteilhaft ausgewählt aus der Gruppe Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erycyloleat, Erucylerucat sowie synthetische, halbsynthetische und natürliche Gemische solcher Ester.

Weitere vorteilhafte Gruppen für die Ölphase umfassen verzweigte und unverzweigte Kohlenwasserstoffe und -wachse, Silikonöle, Dialkylether, gesättigte oder ungesättigte, verzweigte oder unverzweigte Alkohole sowie der Fettsäuretriglyceride, wie z.B. der Triglyceride gesättigter und/oder ungesättigter, verzweigter und/oder unverzweigter Alkancarbonsäuren einer Kettenlänge von 8 bis 24 C-Atomen. Vorteilhaft sind auch 2-Ethylhexylisostearat Octyldodecanol, Isotridecylisononanoat, Isoeicosan, 2-Ethylhexylcocoat, C₁₂₋₁₅-Alkylbenzoat, Capryl-Caprinsäure-Triglycerid, sowie Dicaprylether. Jegliche Mischung aus Ölen und Wachsen kann erfindungsgemäß miteinander kombiniert werden.

Die erfindungsgemäßen Präparate und Formulierungen enthalten vorteilhafterweise einen oder mehrere Emulgatoren, bevorzugt aus folgenden Gruppen: Phospholipide wie Phosphatidylcholin (PC), Phosphatidylglycerol (PG), Dipalmytoyl-Phosphatidylcholin (DPPC), oder die Mischung der Phospholipide sowie hydrierte Phospholipide. Sucroseester in Kombination mit Glycerylstearat und Glycerylstearatcitrat, Glycerylstearat in Kombination mit Ceteareth-20 und/oder Ceteareth-25, Ceteareth-6 in Kombination mit Stearylalkohol, Cetylstearylalkohol in Kombination mit PEG-40 Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30 Stearat, PEG-40 Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40 Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/Dodecylgycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimonium-chloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Cetylpalmitat, Cetylstearylalkohol in Kombination mit PEG-20 Stearat, PEG-30-Stearat, PEG-40-Stearat und/oder PEG-100-Stearat, hydriertes Lecithin sowie mittelkettige Triglyceride. Die vorgenannten Emulgatoren sind bevorzugt und können Bestandteil des Trägersystems sein.

Die Präparate und Formulierungen können Systemstabilisatoren und Konsistenzerhöhende Substanzen enthalten sein wie z.B. Cellulosederivate, Chitosanderivate, Stärke-Derivate, Guarmehl, Agar, Carrageenan-Derivate, Alginate, Polysaccaride.

In einer weiteren Ausführungsform liegen die Präparate und Formulierungen als Gel vor, die neben dem Pandanus-Extrakt und den üblicherweise verwendeten Lösungsmitteln, weiterhin organische Verdickungsmittel enthalten, wie z.B. Gummiarabikum, Xanthangummi, Natriumalginat, Cellulose-Derivate, bevorzugt Methylcellulose, Hydroxymethylcellulose, Hydroxyethylcellulose, Hydroxypropylcellulose, Hydroxypropylmethylcellulose oder anorganische Verdickungsmittel, wie z.B. Aluminiumsilikate, beispielsweise Bentonit oder ein Gemisch aus Polyethylenglykol und Polyethylenglykolstearat oder -distearat. Auch diese Substanzen können Bestandteil des Trägersystems sein.

Die Präparate und Formulierungen können formuliert sein als Gele, Salben, Cremes, Lotion, Lösungen, Pasten, Öle, Suspensionen, Pulver oder halbfeste Formulierungen sowie auch wasserfreie Zubereitungen. Vorzugsweise enthalten die vorgenannten Formulierungen eine Emulsion und weiter bevorzugt eine Mikroemulsion oder eine Nanoemulsion des Pandanusextraktes in einer wässrigen Phase oder einfach in Wasser.

Kosmetische und pharmazeutische Zusammensetzungen und Formulierungen enthalten für den Fachmann bekannte Hilfs- und Zusatzstoffe, dazu gehören Konsistenzgeber, Füllstoffe, Parfum, Farbstoffe, Emulgatoren, Stabilisatoren, zusätzliche Wirkstoffe wie Vitamine, Lichtschutzmittel, Alkohol, Wasser, Salze sowie antimikrobielle oder proteolytische Substanzen, welche zusammen mit der wässrigen Phase oder dem Wasser das Trägersystem bilden können.

Die Präparate können Lipid-Partikel enthalten, in denen die Extrakt-Wirkstoffe transportiert werden. Der Pandanus-Extrakt wirkt durch den hohen Gehalt an Phytolipiden regenerativ auf die Haut-Lipid-Barriere. Bereits durch die Wiederherstellung der Lipidbarriere kann eine Verminderung der Symptomatik erreicht werden. Eine kosmetische oder pharmazeutische Formulierung, welche eine Emulsionsformulierung ist oder eine Emulsion, vorzugsweise eine Mikroemulsion oder eine Nanoemulsion enthält, ist am besten für die Regeneration der Lipidbarriere geeignet.

Weitere Formulierungen ohne hautpflegende Zusatzstoffe wurden auch entwickelt und evaluiert. Aufgrund der hohen Konzentration von Phytolipiden und der starken antientzündlichen Wirkung von Pandanus-Extrakt, ist allein die Wirkung des Extraktes in der Lage, die Hauptsymptomatik bei Neurodermitis, nämlich den Juckreiz, zu unterdrücken. Dies geschieh überraschend um so besser, je kleiner die Pandanus-Extrakt-enthaltenden Öltröpfchen in einer Emulsion sind.

Die Behandlung von Neurodermitis erfolgt meist über die Stufenplan-Therapie:
1. Stufe: Einsatz von fetthaltigen feuchtigkeitshaltenden Stoffen (Emollientia) als Basistherapie, um die Haut-Lipid-Barriere zu regenerieren
2. Stufe: Einsatz von anti-inflammatorischen Substanzen wie Corticosteroiden in der Schubphase, um die Ausschüttung der Entzündingsfaktoren zu unterdrücken.
3. Stufe: bei hohem Schweregrad der Erkrankungen können Calcineurinhemmer wie Tacrolimus eingesetzt werden.

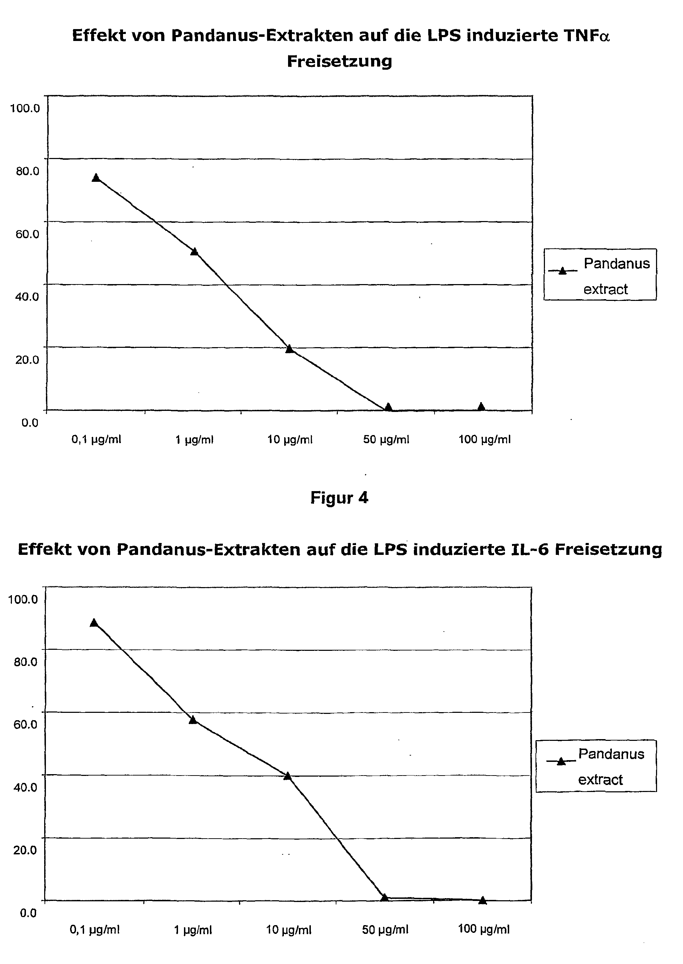

Die vorliegenden Ergebnisse zeigen jedoch überraschenderweise eine unerwartet starke anti-inflammatorische Wirkung des Extraktes. Die IC-50 Werte für die Suppression der Entzündungsfaktoren TNF-α, IL-6 und PGE-2 betragen im Bereich < 10 ug/ml für IL-6 und TNF-Alpha bzw. < 50 ug/ml für PGE2 (Fig. 3-5). Der Pandanus-Extrakt wirkt also dualistisch, indem der Extrakt die Haut-Lipid-Barriere wiederherstellt und die Entzündungsprozesse stark unterdrückt. Insofern verlaufen die Wirkmechanismen des Extraktes ähnlich dem empfohlenen Therapieschema zur Behandlung von immundermatologischen Erkrankungen. Diese unerwartet starke und dualistische Wirkungsweise des Pandanus-Extraktes war überraschend und führt bei dermatologischen und immundermatologischen Hauterkrankungen zu einer deutlich besseren Wirkung als der Fachmann erwartet hätte und zu einer deutlich besseren Wirkung als ähnliche Extrakte, welche für die Pflege und Regeneration der Haut eingesetzt werden wie beispielsweise Orchideenextrakt, Rosenextrakt, Hamamelisextrakt, Aloe Vera Extrakt, Tonkaextrakt, Dinkelextrakt oder ähnliches. Bei Untersuchungen an Probanden mit trockener Haut schnitt von den vorgenannten Extrakten gemäß subjektiver Einschätzungen und Eindrücken der Probanden nach dreiwöchiger und täglich mehrmaliger Anwendung der Pandanus-Extrakt am besten ab. Alle vorgenannten Extrakte wurde als Creme in gleicher Konzentration gemäß Formulierung 6 hergestellt und durch Auftragen und Einreiben von ca. 1 ml Creme pro 4 cm² trockener Hautoberfläche getestet.

Die erfindungsgemäß eingesetzten Extrakte können auf bekannte Art und Weise gewonnen werden, beispielsweise
1) durch die direkte Verpressung des roten Fruchtfleisches, woraus ein ölhaltiges oder öliges Produkt erhalten wird
2) durch eine Extraktion des roten Fruchtfleisches mit organischen Lösungsmitteln insbesondere lipophilen Lösungsmitteln, woraus ebenfalls ein ölhaltiges oder öliges Produkt erhalten wird
3) durch alkoholische Extraktion des roten Fruchtfleisches,
4) durch alkoholische Extraktion der Kerne,
5) durch die direkte Verpressung oder durch die Alkohol-Extraktion des roten Fruchtfleisches zusammen mit den Kernen
6) durch direkte Verpressung des hellen inneren Fruchtanteils, wodurch ein wässriger Saft gewonnen wird oder
7) durch alkoholische Extraktion des getrockneten hellen, inneren Fruchtanteils, wodurch hydrophile Extrakte erhalten werden.

Diese Extrakte können in reiner Form oder aufbereitet als kosmetisches Mittel zur Anwendung bei trockener Haut, Stoffwechselstörungen der Haut, Akne, geschädigter Haut, Alterungserscheinungen sowie zur Glättung der Haut und zur Steigerung der Feuchtigkeitsversorgung der Haut eingesetzt werden.

Ferner können diese Extrakte in reiner Form oder aufbereitet als pharmazeutisches Mittel zur Behandlung und Prophylaxe von Neurodermitis, Dermatitis, oder Akne verwendet werden.

Bei der Anwendung der Extrakte der Spezies *Pandanus conoideus* konnten keinerlei unerwünschte Nebenwirkungen festgestellt werden, so dass die Extrakte als sehr gut verträglich eingestuft werden und für eine Langzeitanwendung, wie z.B. bei Neurodermitis erforderlich, geeignet sind.

Somit betrifft die vorliegende Erfindung die Verwendung eines Extraktes gewonnen aus der Spezies *Pandanus conoideus* zur Herstellung einer topischen Formulierung ohne oder mit einem Trägersystem zur Behandlung und Prophylaxe von Erkrankungen der Haut, wobei die topische Formulierung eine Mikroemulsion oder eine Nanoemulsion mit einer durchschnittlichen Tröpfchengröße von 50nm bis zu 50µm enthält oder aus einer Mikroemulsion oder aus einer Nanoemulsion mit einer durchschnittlichen Tröpfchengröße von 50nm bis zu 50µm besteht. Bei den Erkrankungen der Haut handelt es sich um Neurodermitis, Dermatitis, Psoriasis, oder Akne. Falls die topische Formulierung ein Trägersystem enthält, so enthält die topische Formulierung ein Trägersystem in Form einer Öl-in-Wasser-Emulsion oder einer Wasser-in-Öl-Emulsion und bevorzugt einer Öl-in-Wasser-Emulsion.

Der Begriff Trägersystem umfasst hierbei alle Komponenten, die nicht durch Extraktion aus der Spezies *Pandanus Conoideus* gewonnen wurden. Somit werden grundsätzlich alle Bestandteile inklusive des Wassers, welche nicht zu dem Pandanusextrakt gehören als Trägersystem bezeichnet. Vorzugsweise ist der Pandanusextrakt gelöst, emulgiert und/oder dispergiert in einem flüssigen, halbfesten oder festen Trägersystem. Beispiele für geeignete Trägersysteme sind Flüssigkeiten wie Wasser oder wässrige Pufferlösungen, physiologisch verträgliche organische Lösungsmittel wie Ethanol oder Kombinationen davon, Öl-Wasser-Emulsionen, Wasser-Öl-Emulsionen, Fette, Polyethylenglykole, Propylenglykole, Glycerin, Emulgatoren oder Kombinationen davon sowie andere in pharmazeutischen und kosmetischen Formulierungen verwendete Träger- oder Hilfsstoffe sowie Penetrationsverstärker und Emulgatoren. Diese Trägersysteme vermitteln vorzugsweise eine gezielte Wirkstoffabgabe an die Hautzellen bzw. modulieren die Absorption durch die Hautzellen, wodurch eine länger anhaltende Wirkung des Pandanusextraktes erzielt werden kann. Insbesondere wird durch die Formulierung mit einem Trägersystem eine stabile Formulierung erhalten, die besonders für topische Präparate zur Behandlung von Hautkrankheiten vorteilhaft ist.

Das erfindungsgemäße topische Präparat enthält vorteilhafterweise einen oder mehrere Penetrationsverstärker. Der Begriff Penetrationsverstärker umfasst dabei alle Substanzen, die unabhängig von ihrer Wirkweise, eine verbesserte Penetration von Wirkstoffen durch die Haut bewirkt.

Die Penetrationsverstärker werden ausgesucht aus der Gruppe der Fettalkohole, wie aliphatische Alkohole, Decanol, Laurylalkohol (Dodecanol), Linolenylalkohol, Nerolidol, 1-Nonanol, n-Octanol, Oleylalkohol, weiterhin aus der Gruppe der Fettsäureester, wie Essigsäure-n-butylester, Cetyl Laktat, Decyl N,N-dimethylaminoacetat, Decyl *N,N-* dimethylaminoisopropionat, Diethylenglycol Oleat, Diethylsebacat, Dodecyl *N,N-* dimethylaminoacetat, Dodecyl (*N,N-*dimethylamino)-Butyrat, Dodecyl *N,N-* dimethylaminoisopropionat, Dodecyl 2-(dimethylamino)propionat, EO-5-oleylester, Ethylacetat, Ethylacetoacetat, Ethylpropionat, Glyzerolmonoether, Glyzerolmonolaurat, Glyzerolmonolinoleat, Isopropylisostearat, Isopropyllinoleat, Isopropylmyristat, Isopropylmyristat / Fettsäureglycerid Monoglycerid Kombination, Isopropylmyristat/-ethanol/L-Milchsäure (87:10:3) Kombination, Isopropylpalmitat, Methylacetat, Methylcaprate, Methyllaurat, Methylpropionat, Methylvalerat, 1-Monocaproylglycerol, Monoglyceride (Mittlere Kettenlänge), Nikotinester (Benzyl), Octylacetat, Octyl N,N-dimethylaminoacetat, Oleyloleat, n-Pentyl N-Acetylprolinat, Propylenglycolmonolaurat, Sorbitandilaurat, Sorbitandioleat, Sorbitanmonolaurat, Sorbitanmonooleat, Sorbitantrilauratem, Sucrose Kokusnuss Fettsäure Mixturen, Sucrosemonolaurat, Sucrosemonooleat, Tetradecyl *N,N*dimethylaminoacetat.

Weiterhin vorteilhaft sind Penetrationsverstärker aus der Gruppe der Fettsäuren, wie Alkansäuren, Caprinsäuren, zweiwertige Säuren, Ethyloctadecanoinsäuren, Hexansäuren, Milchsäuren, Laurinsäuren, Linoelaidinsäuren, Linoleinsäuren, Linolensäuren, Neodecanoinsäuren, Pelargonsäuren, Propionsäuren sowie Vaccensäuren, weitere vorteilhafte Penetrationsverstärker finden sich in der Gruppe der Fettsäureester, wie α-Monoglycerylether, EO-2-Oleylether, EO-5-Oleylether, EO-10-Oleylehter, Ether Derivate von Polyglycerol und Alkoholen (1-O-Dodecyl-3-O-Methyl-2-0-(2',3'-dihydroxypropyl(glycerol). Weiter vorteilhaft sind L-α-Aminosäuren, Lecithin, Phospolipide, Saponin/Phospholipide, Natriumdeoxycholat, Natriumtaurocholat, Natriumtauroglycocholat, Säurephosphatasen, Calonasen, Orgelsen, Papain, Phospholipase A-2, Phospholipase C, Triacylglycerolhydrolase, Acetamid-Derivate, acyclische Amide, N-Adamantyl n-Alkanamid, Clofibrinsäureamid, *N,N*-Didodecylacetamid, Di-2-Ethylhexylamin, Diethylmethylbenzamid, *N,N*Diethyl-m-toluamid, *N,N*-Dimethyl-m-toluamid, Ethomeen S12 [bis-2(2-hydroxyethyl)oleylamine], Hexamethylenlauramid, Laurylamin (Dodecylamin), Octylamid, Oleylamin, Ungesättigte cyclische Harnstoffe, Harnstoff, β-und γ-cyclodextrin Komplexe, Hydroxypropylmethylcellulose, Liposome, Naphthalendiamiddiimid, Naphthalenediesterdiimid, Macrocyclische Lacton, Ketone und Anhydride.

Aus der Gruppe der Tenside ist der Einsatz von Penetrationsverstärken besonders vorteilhaft, wie Polyoxyethylen-(4)-lauryl-ether, Polyoxyethylen-(23)-laurylether, Polyoxyethylen-(2)-cetylether, Polyoxyethylen-(1 0)-cetylether, Polyoxyethylen-(20)-cetylether, Polyoxyethylen-(2)-stearylether, Polyoxyethylen-(20)-stearylether, Polyoxyethylen-(2)-oleylether, Polyoxyethylen-(20)-oleylether, Cetyltrimethylammoniumbromid, Empicol ML26/F, HCO-60, Hydroxypolyethoxydodecan, Ionische Tenside (ROONa, ROSO₃Na, RNH₃Cl, R=8-16), Lauroylsarcosin, Nonoxynol, Octoxynol, Phenylsulfonat, Pluronic F127, Pluronic L62, Pluronic F68 (Polyoxyethylen-polyoxypropylen block copolymer), Polyoleat, Rewopal HV10, Natriumlaurat, Natriumlaurylsulfat (Natriumdodecylsulfat), Natriumoleat, Span 20, Span 40, Span 85, Synperonic NP, Triton X-100, Tween-20, Tween-40, Tween-60, Tween-80 sowie Tween-85.

Weiterhin vorteilhaft sind aliphatische Thiole, Alkyl *N,N*-dialkyl-substituierte aminoacetat, Anisöl, anticholigerne Substanzen, Ascaridol, Biphasische Derivative, Bisabolol, Kardamomöl, 1-Carvon, Chenopodiumöl, 1,8 Cineole (Eukalyptusöl), Lebertran (Fettsäureextrakt), 4-Decyloxazolidin-2-one, Dicyclohexylmethylaminoxid, Diethylhexadecylphosphonat, Diethylhexadecylphosphoramidat, *N,N*-Dimethyldodecylamin-*N*-oxid, 4,4-Dimethyl-2-undecyl-2-oxazolin, N-Dodecanoyl-L-aminosäuremethylester, 1,3-Dioxacycloalkan (SEPAs), Dithiothreitol, Eucalyptol (cineol), Eugenol,Kräuterextrakte, Lactam N-Essigsäureester, N-Hydroxyethalaceamid, 2-Hydroxy-3-oleoyloxy-1-pyroglutamyloxypropan, Menthol, Menthon, Morpholin Derivate, N-Oxide, Nerolidol, Octyl-β-D-(thio)glucopyranosid, Oxazolidinon, Piperazin Derivate, polare Lipide, Polydimethylsiloxan, Poly [2-(methylsulfinyl)ethylacrylat], Polyrotaxan, Polyvinylbenzyldimethylalkylammoniumchlorid, Poly(N-vinyl-N-methylacetamid), Prodrugs, Salzlösungen, Natriumpyroglutaminat, Terpene und Azacyclo Ringverbindungen, Vitamin E (α-tocopherol) sowie Ylang-ylang Öl.

Weiterhin vorteilhaft sind Penetrationsverstärker aus der Gruppe der *N*-methyl Pyrrolidone, wie *N*-Cyclohexyl-2-pyrrolidon, 1-Butyl-3-dodecyl-2-pyrrolidon, 1,3-Dimethyl-2-imidazolikinon, 1,5 Dimethyl-2-pyrrolidon, 4,4-Dimethyl-2-undecyl-2-oxazolin, 1-Ethyl-2-pyrrolidon, 1-Hexyl-4-methyloxycarbonyl-2-pyrrolidon, 1-Hexyl-2-pyrrolidon, 1-(2-Hyroxyethyl)pyrrolidinon, 3-Hydroxy-N-methyl-2-pyrrolidinon, 1-Isopropyl-2-undecyl-2-imidazolin, 1-Lauryl-4-methyloxycarbonyl-2-pyrrolidon, N-Methyl-2-pyrrolidon, Poly(N-vinylpyrrolidon, Pyroglutamicsäureester und 2-Pyrrolidon (2-pyrrolidinon).

Aus der Gruppe der ionischen Verbindungen ist der Einsatz von Penetrationsverstärken besonders vorteilhaft, wie Ascorbate, Kalziumthioglycolat, Cetyltrimethylammonioumbromid, 3,5-Diiodosalicylatenatrium, Lauroylcholiniodid, 5-Methoxysalicylatenatrium, Monoalkylphosphat, 2-PAM chlorid, 4-PAM chlorid (Derivative von *N*-methylpicoliniumchlorid, Natriumcarboxylate, Natriumhyaluronate, Natriumlaurylsulfat (Natriumdodecylsulfat). Weiterhin auch Dimethylsulfoxid und verwandte Substanzen, wie cyclische Sulfoxid, Decymethylsulfoxid und 2-Hydroxyundecylmethylsulfoxid.

Die Penetrationsverstärker werden weiterhin ausgesucht aus der Gruppe der Lösungsmittel und verwandten Substanzen, wie Acetamide, Aceton, n-Alkane (Kettenlänge zwischen 7 und 16), Alkanole, Diole, kurzkettige Fettsäuren, Cyclohexyl-1,1-dimethlethanol, Dimethylacetamid, Dimethylformamid, Ethanol, Ethanol/d-limonen Kombinationen, 2-ethyl-1,3-hexanediol, Ethoxydiglycol, Glycerol, Glycol, Laurylchlorid, Limonenem *N*-Methylformamid, 2-Phenylethanol, 3-Phenyl-1-propanol, 3-Phenyl-2-propen-1-ol, Polyethylenglycol, Polyoxyethylensorbitanmonoester, Polypropylenglycol, Tridecanol, Propylenglycol, Span 20, Squalen, Triacetin, Trichloroethanol, Trifluoroethanol, Trimethylenglycol, Xylen.

Eine weitere vorteilhafte Gruppe der Penetrationsverstärker umfasst die Azone, wie *N*-Acyl-hexahydro-2-oxo-1H-azepin, *N*-Alkyl-dihydro-1,4-oxazepin-5,7-dion, N-Alkylmorpholin-2,3-dion, N-Alkylmorpholin-3,5-dion, Azacycloalkan Derivate (Ketone und Thione), Azacycloalkenon Derivate, 1-[2-(Decylthio)ethyl]azacyclopentan-2-one (HPE-101), *N-*(2,2.Dihydroxyethyl)dodecylain, 1-Dodecanoylheyxahydro-1-H-azepin, 1-Dodecylazacycloheptan-2-one (Azon oder Laurocapram), N-Dodecyldiethyanolamin, *N*-Dodecyl-hexahydro-2-thio-1H-azepin, N-Dodecyl-N-(2-methoxyethyl)acetamid, *N*-Dodecyl-*N*-(2-methoxyethyl)isobutyramid, N-Dodecyl-piperidin-2-thion, N-Dodecyl-2-piperidinon, *N*-Dodecylpyrollidin-3,5-dion, N-Dodecylpyrrolidin-2-thion, N-Dodecyl-2-pyrrolidon, 1-Farnesylazacycloheptan-2-one, 1-Farnesylazacyclopentan-2-one, 1-Geranylazacycloheptan-2-one, 1-Geranylazacyclopentan-2-one, Hexahydro-2-oxo-azepine-1-Essigsäureester, *N-*(2-hydroxyethyl)-2-pyrrolidon, 1-Laurylazacycloheptan, 2-(1-Nonyl)-1,3-dioxolan, 2-(1-Nonyl)-1,3-dioxolan, 1-*N*-Octylazacyclopentan-2-one, *N*-(1-Oxododecyl)-hexahydro-1H-azepin, *N*-1(1-Oxododecyl)-morpholin, 1-Oxohydrocarbylsubstituierte azacyclohexane, *N*-(1-Oxotetradecyl)-hexahydro-2-oxo-1H-azepin sowie N-(1-Thiododecyl)-morpholin. Sämtliche vorgenannte Penetrationsverstärker können Bestandteil des Trägersystems sein.

Zudem betrifft die vorliegende Erfindung die Verwendung von Extrakten der *Pandanus conoideus* zur Herstellung eines pharmazeutischen Mittels zur Behandlung und Prophylaxe von Neurodermitis, Dermatitis, Akne und Psoriasis. Die als pharmazeutisches Mittel eingesetzten Formulierungen können auch als kosmetische Zubereitung dienen und bei trockener Haut, Alterungserscheinungen der Haut und zur Feutigkeitsversorgung der Haut eingesetzt werden.

Ferner offenbart die vorliegende Anmeldung Verfahren zur Gewinnung der Extrakte wie beispielsweise eines lipophilen Extraktes aus dem roten Fruchtfleisch der Früchte der Spezies *Pandanus conoideus,* der durch die direkte Verpressung, d.h. Kaltpressung des abgetrennten roten Fruchtfleisches bevorzugt ohne jegliche chemische Zusätze bei Temperaturen vorzugsweise unterhalb von 40°C oder durch Extraktion des abgetrennten zerkleinerten roten Fruchtfleisches mittels organischer Lösungsmittel bei Temperaturen vorzugsweise unterhalb von 40°C gewonnen wird. Die Extraktionen können grundsätzlich bei Temperaturen zwischen 4°C und 100°C durchgeführt werden. Insbesondere bevorzugt ist jedoch die Kaltpressung unterhalb von 40°C da zum einen festgestellt worden ist, dass beim Kochen des roten Fruchtfleisches in Wasser oder einem organischen Lösungsmittel wichtige bioaktive Stoffe wie beispielsweise Tocopherole und andere Vitamine, Phytosterole etc. zerstört werden. Darüber hinaus wird ein durch Kochen hergestellter Extrakt sehr schnell ranzig. Durch die bevorzugte Kaltpressung erhält man überraschenderweise Extrakte, vorrangig Öle, welche mindestens ein Jahr und wahrscheinlich noch deutlich länger stabil sind. Die bisherigen Stabilitätsdaten zeigen eine Stabilität von zwei Jahren von Proben, welche bisher zwei Jahre lang bei 4 °C Jahr gelagert worden sind. Die Stabilitätsprüfung wird weiter fortgesetzt und es werden noch deutlich längere Stabilitäten erwartet.

Eine Temperaturerhöhung während des Extraktionsverfahrens oder des Verarbeitungsverfahrens des roten Fruchtfleisches über 40°C führt überraschenderweise zu einer Verringerung der Stabilität und Lagerstabilität des erhaltenen Extraktes. Je länger die Temperatur während der Extraktion des roten Fruchtfleisches über 40°C liegt und je höher die Temperatur während des Herstellungsprozesses des Öles aus dem rotem Fruchtfleisch über 40°C liegt, desto niedriger wird die Stabilität des Extraktes. Somit ist es wichtig, bei dem gesamten Herstellungsverfahrens des Öls aus dem roten Fruchtfleisch darauf zu achten, dass die Extraktions- und Verarbeitungsbedingungen eine Temperatur von 60°C, bevorzugt 50°C und insbesondere bevorzugt 40°C nicht überschreiten und im Idealfall der gesamte Prozess der Extraktion und Ölgewinnung bei Raumtemperatur durchgeführt wird.

Für die Extraktion werden bevorzugt Hexan, Heptan, Cyclohexan, Chloroform, Methylenchlorid, Essigester, Diethylether, Petrolether, tert-Butylmethylether, THF, Methanol, Ethanol, Propanol, iso-Propanol, Butanol, iso-Butanol, sec-Butanol, Aceton und Mischungen dieser Lösungsmittel verwendet. Nach der Extraktion wird das Lösungsmittel vorzugsweise wieder entfernt und recyclisiert, um für weitere Extraktionen erneut verwendet zu werden. Das aufkonzentrierte Öl enthält die gewichtsbeeinflussenden Bestandteile des extrahierten Fruchtfleisches.

Ein weiteres bevorzugtes Extraktionsverfahren ist eine Alkohol und/oder Alkohol-Wasser-Extraktion des Fruchtfleisches samt Kerne, indem zuerst das rote Fruchtfleisch samt Kerne bei Temperaturen unterhalb von vorzugsweise 40°C zermahlen, mit Alhokol und/oder -Wasser-Mischung unter Rühren vermischt und danach gepresst und filtriert werden. Vor dem Zermahlen kann Fruchtfleisch und Kerne auch getrocknet werden. Die zerriebenen Kerne samt verbliebenem zerriebenem Fruchtfleisch werden danach analog des Verfahrens zur Alkohol-und/oder Alkohol-Wasser-Extraktion des frischen Materials behandelt.

Ein weiteres Verfahren der vorliegenden Anmeldung betrifft die Herstellung eines hydrophilen Extraktes aus den abgetrennten Kernen der Frucht der Spezies *Pandanus conoideus* durch Zerkleinerung der Kerne bei Temperaturen unterhalb von vorzugsweise 40°C und Extraktion der zerkleinerten Kerne mit einem hydrophilen Lösungsmittel bei Temperaturen unterhalb von vorzugsweise 40°C. Auch hier gilt wie bei den anderen Extraktionen, dass die Extraktionen grundsätzlich bei Temperaturen zwischen 4°C und 100°C durchgeführt werden können, vorzugsweise jedoch Temperaturen von 60°C und insbesondere bevorzugt Temperaturen von 40°C nicht überschritten werden sollten.

Als hydrophiles Lösungsmittel werden vorzugsweise Wasser, Aceton, Tetrahydrofuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion und Vereinigung der hydrophilen Extrakte, wird das Lösungsmittel wieder entfernt und erneut verwendet. Diese Schritte sollten vorzugsweise bei Temperaturen unterhalb von 40°C durchgeführt werden.

Ein weiteres Verfahren der vorliegenden Anmeldung betrifft die Herstellung eines hydrophilen Extraktes aus dem weißen Fruchtfleisch der Früchte der Spezies *Pandanus conoideus* durch eine direkte Verpressung des abgetrennten weißen Fruchtfleisches mit oder ohne Zusätze wie z.B. Zusatz von Antioxidantien bei Temperaturen vorzugsweise unterhalb von 40°C oder durch Trocknung und Zerkleinerung des abgetrennten weißen Fruchtfleisches bei Temperaturen unterhalb von vorzugsweise 40°C und anschließender Extraktion des getrockneten zerkleinerten weißen Fruchtfleisches mittels hydrophiler Lösungsmittel.

Als hydrophiles Lösungsmittel werden vorzugsweise Wasser, Aceton, Tetrahydröfuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion des weißen bzw. hellen Fruchtfleisches und Vereinigung der hydrophilen Extrakte, wird das Lösungsmittel wieder entfernt und kann erneut verwendet werden. Alle Extraktionsschritte sollten vorzugsweise bei Temperaturen unterhalb von 40°C durchgeführt werden. Der aufkonzentrierte Extrakt enthält die pharmakologisch aktiven Bestandteile aus dem weißen Fruchtfleisch der *Pandanus conoideus.*

Die Frucht der *Pandanus conoideus* besteht aus einem inneren hellen Fruchtanteil und einem roten, kernhaltigen und ölhaltigen äußeren Fruchtanteil. Die vorliegende Anmeldung offenbart 7 mögliche Extrakte, welche aus dieser Frucht gewonnen werden können. Diese Extrakte können alleine als auch kombiniert angewendet werden, vorzugsweise als wässrige Lösung, als Öl oder als Feststoff.

Zur Gewinnung der bioaktiven Extrakte werden die Früchte gereinigt und die rote, äußere Fruchtschicht von dem weißen, hellen, inneren Fruchtanteil abgetrennt. Die verschiedenen Extrakte können auf verschiedene Arten erhalten werden, wie beispielsweise in den Beispielen 1 - 7 offenbart ist.

Sämtliche Arbeitsschritte werden bevorzugt bei Temperaturen von 5 - 80°C, weiter bevorzugt bei 10°C bis 60°C, weiter bevorzugt bei 15 - 45°C, noch weiter bevorzugt bei 17 - 40°C und insbesondere bevorzugt bei 19°C - 38°C durchgeführt. Bevorzugt ist ferner, wenn die Arbeitsschritte bei Temperaturen unterhalb von 60°C, bevorzugt unterhalb von 50°C und insbesondere bevorzugt unterhalb von 40°C ausgeführt werden.

Ein vereinfachtes Verfahren zur Gewinnung eines Extraktes aus dem roten Fruchtfleisch besteht darin, dass zuerst das rote Fruchtfleisch von den Kernen zu trennen, danach zu zerkleinern und mit einem organischen Lösungsmittel mehrmals zu extrahieren. Verwendet man für die Extraktion Ethanol, so kann dieser alkoholische Extrakt direkt als pharmazeutisches oder kosmetisches Mittel eingesetzt werden. Dieses Verfahren ist insbesondere für die Verwendung von physiologisch verträglichen Lösungsmitteln geeignet und vorzugsweise für Alkohole wie Ethanol, Isopropanol und insbesondere für Ethanol.

Ein bevorzugtes Verfahren zur Gewinnung eines Extraktes aus dem roten Fruchtfleisch zusammen mit den Kernen besteht darin, dass zuerst das rote Fruchtfleisch samt den Kernen zu zerkleinern und mit einem organischen Lösungsmittel mehrmals zu extrahieren. Verwendet man für die Extraktion Ethanol, so kann dieser alkoholische Extrakt direkt als pharmazeutisches oder kosmetisches Mittel eingesetzt werden. Dieses Verfahren ist insbesondere für die Verwendung von physiologisch verträglichen Lösungsmitteln geeignet und vorzugsweise für Alkohole wie Ethanol, Isopropanol und insbesondere für Ethanol. Selbstverständlich kann der Extrakt auch aus dem getrockneten roten Fruchtfleisch samt den Kernen nach dem gleichen Verfahren hergestellt werden.

Ein vereinfachtes Verfahren zur Gewinnung eines alkoholischen Extraktes aus dem hellen Fruchtfleisch besteht darin, das helle weiße innere Fruchtfleisch zu trocknen, bevorzugt an der Luft, danach zu zerkleinern und mit einem hydrophilen Lösungsmittel mehrmals zu extrahieren. Verwendet man für die Extraktion Ethanol oder Ethanol-Wasser-Gemische, so kann dieser alkoholische Extrakt direkt als Medizin eingesetzt werden.

Eine weitere Möglichkeit besteht daran, das innere helle Fruchtfleisch kalt zu pressen (bevorzugt T<40°C). Der erhaltene wäßrige Extrakt kann in der vorliegenden Form als pharmazeutisches oder kosmetisches Mittel verwendet werden.

Erfolgt die Extraktion der abgetrennten und zerkleinerten Kerne mittels Ethanol, so kann auch dieser ethanolische Extrakt unmittelbar als pharmazeutisches oder kosmetisches Mittel eingesetzt werden.

Somit betrifft die vorliegende Anmeldung auch insbesondere ethanolische Extrakte, welche unmittelbar bei schonenden Bedingungen (vorzugsweise T<40°C) aus dem roten, aus dem hellen bzw. weißen Fruchtfleisch sowie aus den Kernen gewonnen wurden. Die gewonnene alkoholische Lösung kann eingeengt werden und kann zur Zubereitung einer ethanolischen Lösung oder eines Feststoffs nach Trocknung dienen, wobei der Feststoff als aktive Komponente üblichen Formulierungen zugesetzt werden kann.

Ein bevorzugtes Extraktionsverfahren für *Pandanus conoideus* umfasst beispielsweise die folgenden Schritte:
1) Trennung der Frucht in helles, weißes inneres Fruchtfleisch sowie in die rote Schicht wo das rote Fruchtfleisch samt den Kernen sich befinden.
2) Kaltpressung oder Extraktion der roten Schicht mit organischen Lösungsmitteln um die ölige Fraktion zu gewinnen, und
3) Trennung der roten Schicht in rotes Fruchtfleisch und Kerne.
4) Kaltpressung des roten Fruchtfleisches, um die Ölfraktion zu gewinnen, und
5) Extraktion des Rückstandes des roten Fruchtfleisches mit Alkohol (z.B. Methanol, Ethanol, Propanol, iso-Propanol, Butanol); oder
6) Extraktion des Öls direkt aus dem roten Fruchtfleisch mit Alkohol oder anderen organischen Lösungsmitteln,
7) Extraktion der Kerne mit hydrophilen Lösungsmitteln,
8) Kaltpressen des weißen hellen Fruchtfleisches, um Saft zu gewinnen, oder
9) optional Extraktion des weißen hellen Fruchtfleisches mit hydrophilen Lösungsmitteln, und/oder
10) Trocknung des weißen hellen Fruchtfleisches, und
11) anschließende Extraktion des weißen hellen Fruchtfleisches mit hydrophilen Lösungsmitteln.

Ähnliche Extraktionsverfahren können wie folgt beschrieben werden und umfassen zumindest einige der folgenden Schritte:
a) Trennung der Frucht in helles weißes inneres Fruchtfleisch sowie rotes Fruchtfleisch samt den Kernen,
b) Trennung des roten Fruchtfleisch von den Kernen,
c) Kaltpressung oder Extraktion mit organischen Lösungsmittel des roten Fruchtfleisches zusammen mit den Kernen
d) Kaltpressung des roten Fruchtfleisches, und
e) Zerkleinerung und/oder Homogenisierung des roten Fruchtfleisches, und
f) Extraktion des Saftes aus dem roten Fruchtfleisch sowie des zerkleinerten oder homogenisierten roten Fruchtfleisches mit Alkoholen oder anderen organischen lipophilen Lösungsmitteln, und
g) Sammlung und Einengung der lipophilen Extrakte, oder
h) Zerkleinerung der Kerne und
i) Extraktion der zerkleinerten Kerne mit hydrophilen Lösungsmitteln, und
j) Sammlung und Einengung der hydrophilen Extrakte, oder
k) Kaltpressen des weißen hellen inneren Fruchtfleisches, oder Zerkleinerung und/oder Homogenisierung des getrockneten weißen hellen Fruchtfleisches, und
l) Extraktion des zerkleinerten oder homogenisierten getrockneten weißen hellen Fruchtfleisches mit hydrophilen Lösungsmitteln, und
m) Sammlung und Einengung der hydrophilen Extrakte.

Ein Extrakt oder mehrere Extrakte aus der *Pandanus conoideus* wird/werden vorzugsweise zur Herstellung einer pharmazeutischen, dermatologischen oder kosmetischen Zusammensetzung verwendet, welche den Extrakt oder die Extrakte aus *Pandanus conoideus* zusammen mit mindestens einem pharmakologisch oder kosmetisch verträglichen Hilfsstoff, Trägerstoff und/oder Lösungsmittel enthält.

Die vorliegende Anmeldung offenbart Verfahren, nach denen aus der Frucht der Pflanze *Pandanus conoideus* verschiedene Extrakte gewonnen werden können, je nachdem, wie die Extraktion durchgeführt wird. Natürlich sei angemerkt, dass rotes Fruchtfleisch und helles weißes Fruchtfleisch auch gemeinsam extrahiert werden können und eine vorherige Trennung nicht zwingend ist. Gleiches gilt für das rote Fruchtfleisch und die Kerne. Diese Pandanus-Extrakte zeigen alle vergleichbare pharmakologische sowie kosmetische Aktivität, d.h. alle Extrakte sind gleichermaßen zur Prophylaxe und Behandlung von Neurodermitis, Dermatitis, Ekzemen, Psoriasis, Stoffwechselstörungen der Haut, Akne und geschädigter Haut geeignet.

Auch in Bezug auf die kosmetischen Anwendungen zeigen alle Pandanus-Extrakte eine vergleichbare Wirksamkeit insbesondere bei trockener Haut, Stoffwechselstörungen der Haut, Akne, geschädigter Haut, Alterungserscheinungen der Haut, Falten sowie Glättung der Haut und zur Steigerung der Feuchtigkeitsversorgung der Haut.

Somit ist es erfindungsgemäß bevorzugt, zwei oder auch mehrere Extrakte miteinander zu mischen oder zu kombinieren und zur Behandlung der vorgenannten Indikationen einzusetzen. Demnach ist es bevorzugt, einen Extrakt aus dem roten Fruchtfleisch mit einem Extrakt aus den Kernen und/oder mit einem Extrakt aus dem hellen weißen Fruchtfleisch zu mischen.

Genauso kann auch ein Extrakt aus den Kernen mit einem Extrakt aus dem hellen weißen Fruchtfleisch und/oder mit dem Saft nach Kaltpressung des hellen weißen Fruchtfleisches und/oder mit einem Extrakt aus dem roten Fruchtfleisch und/oder mit einem Extrakt aus dem nach Kaltpressung des roten Fruchtfleisches erhaltenen Saft bzw. den Bestandteilen des Saftes nach dessen Trocknung hergestellt werden.

Ein Extrakt aus dem weißen getrockneten hellen Fruchtfleisch und/oder aus dem durch Kaltpressung des weißen hellen Fruchtfleisches gewonnenen Saft kann kombiniert werden mit einem Extrakt aus den Kernen und/oder einem Extrakt aus dem roten Fruchtfleisch und/oder mit einem aus dem Kaltpressung des roten Fruchtfleisches erhaltenen Öl bzw. den Bestandteilen des Öles nach dessen Trocknung.

Durch derartige Kombinationen kann das Wirkspektrum der Kombination von Extrakten bzw. des diese Kombination von Extrakten enthaltende pharmazeutische und kosmetische Mittel verbreitert und die Aktivität auch teilweise noch gesteigert werden.

Die Herstellung der Extrakte aus dem roten Fruchtfleisch oder aus den abgetrennten Kernen oder aus dem roten Fruchtfleisch samt den Kernen oder aus dem hellen, inneren Anteil der Frucht kann auch durch Extraktion mit überkritischen Fluiden (Kohlendioxid, Propan, Butan), vorzugsweise mit Kohlendioxid, mit oder ohne zusätzlichem Einsatz von Schleppmitteln (Methanol, Ethanol) durchgeführt werden. Mittels der vorgenannten Fluide lassen sich bevorzugt lipophile Extrakte erhalten, wobei durch gleichzeitige Verwendung von polaren Schleppmitteln auch weniger lipophile und eher polare bis hydrophile Extrakte gewonnen werden können. Somit werden bei der Extraktion des roten Fruchtfleisches bevorzugt keine polaren Schleppmittel eingesetzt, wohingegen die Verwendung von polaren Schleppmitteln bei der Extraktion der Kerne und des inneren hellen Fruchtfleisches bevorzugt ist.

Die durch Extraktion mit überkritischen Fluiden (Kohlendioxid, Propan, Butan) vorzugsweise mit Kohlendioxid gewonnenen Extrakte können natürlich einzeln oder in Kombination miteinander als auch in Kombination mit den nicht durch überkritische Fluide erhaltenen Extrakte eingesetzt werden. Zur Gewinnung der Extrakte werden vorzugsweise folgende Extraktionsverfahren angewendet: Ethanol-Wasser-Extraktion, Hexan-Extraktion und superkritische CO₂-Extraktion. Unter dem Begriff Extrakt soll sowohl die flüssige Phase nach einer einmaligen Extraktion als auch die kombinierten flüssigen Phasen von mehreren Extraktionen verstanden werden. Zudem soll der Begriff Extrakt auch die eingeengte flüssige Phase umfassen, bei welcher das Lösungsmittel teilweise, weitgehend oder vollständig entfernt worden ist, so dass Extrakt auch ein fester Rückstand bedeuten kann, der nach Entfernung des Lösungsmittels vorzugsweise unter vermindertem Druck und nach Trocknung der festen Bestandteile erhalten worden ist wie beispielsweise ein Lyophilisat.

Die hierin beschriebenen Extrakte können alleine oder in Kombination mit anderen erfindungsgemäßen Extrakten und/oder in Kombination mit weiteren Wirkstoffen wie beispielsweise anti-inflammatorischen Wirkstoffen so wie sie gewonnen wurden oder in Form pharmazeutischer oder kosmetischer Formulierungen und Zusammensetzungen zur Behandlung und Prophylaxe von Neurodermitis, Dermatitis, Psoriasis, und Akne eingesetzt werden.

Das pharmazeutische oder kosmetische Mittel enthaltend einen Extrakt oder mehrere Extrakte aus *Pandanus conoideus* kann mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt werden. Solche Darreichungsformen sind beispielsweise Tabletten, Minitabletten, Mikrotabletten, Filmtabletten, Schichttabletten, Dragees, Kapseln, Mikrokapseln, Mikropellets und Pellets, Pillen, Granulaten, Pulver, Lösungen, Tropfen, Säfte, Suspensionen, Suppositorien, Klysmen, Emulsionen, Dispersionen, Cremes, Gele, Salben, Sirup oder Depotformen. Bevorzugt sind topische Formulierungen wie beispielsweise Salben, Gele, Cremes, Puder, Emulsionen und dergleichen.

Derartige Zubereitungen sind unter anderem für die intravenöse, intraperitoneale, intramuskuläre, subkutane, orale, rektale, transdermale, topikale, intradermale, intragastrale, intrakutane, intranasale, intrabuccale, perkutane oder sublinguale Verabreichung geeignet, wobei die topische Anwendung natürlich bevorzugt ist. Die bevorzugten Formulierungen für die äußere oder topische Anwendung können neben dem Extrakt oder dem Extraktgemisch aus der Spezies *Pandanus conoideus* noch weitere Bestandsteile wie beispielsweise Lecithin, native Öle, Glycerin, Ethylenglykol, 1,3-Propandiol, tert-Butylalkohol, Propylenglykol, Polyethylenglykol, Benzylalkohol, Benzylaceton, Benzaldehyd, Phenylethylalkohol, 2-Phenylacetat, Benzylalkohol, Phenylethylalkohol und gereinigtes Wasser enthalten.

Den hierin beschriebenen Formulierungen können ferner noch übliche Geruchsstoffe bzw. Parfüms, Farbstoffe, Konservierungsstoffe, viskositätserhöhende Stoffe und/oder UV-Filter zugesetzt werden. Diese Zusätze dienen insbesondere zur Erhöhung der Farb-, Licht- und/oder Parfümstabilität der Formulierungen bzw. der Haltbarkeit oder Konsistenzgebung. Derartige Zusatzstoffe sind insgesamt in nicht mehr als 10 Gew.-%, bevorzugt 8 Gew.-%, weiter bevorzugt 6 Gew.-% und insbesondere bevorzugt 4 Gew.-% in der kosmetischen oder pharmazeutischen Formulierung vorhanden.

Unter kosmetischer und/oder dermatologischer Formulierungen sind insbesondere Hautcremes, Hautlotionen, Emulsionen, Salben, Gele, Öle sowie Balsame und sämtliche andere zur topischen Anwendung geeignete Formulierungen zu verstehen.

Orale Formulierungen wie beispielsweise Tabletten können beispielsweise durch Mischen der/des Extrakte(s) mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talkum.

Entsprechend können Dragees durch Überziehen von analog den Tabletten hergestellten Kernen mit üblicherweise in Drageeüberzügen verwendeten Mitteln, beispielsweise Polyvinylpyrrolidon oder Eudragit, Schellack, Gummiarabicum, Talkum, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem Extrakt können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Den Extrakt enthaltende Kapseln können beispielsweise hergestellt werden, indem man den Wirkstoff mit einem inerten Träger wie Milchzucker oder Sorbit mischt und in Gelatinekapseln einkapselt.

Der ölhaltige oder ölige Extrakt kann auch direkt in Weichgelatine-Kapseln verarbeitet werden.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Pulver als auch Tabletten können zu 5 bis 95% aus einem derartigen Träger bestehen.

Ferner können den pharmazeutischen oder kosmetischen Mitteln und Zusammensetzungen noch Sprengmittel, Farbstoffe, Geschmacksstoffe und/oder Bindemittel zugesetzt werden.

Ferner ist bevorzugt, die Extrakte mit pharmazeutischen, kosmetischen Mitteln sowie auch mit den anti-inflammatorischen Wirkstoffen zu kombinieren wie beispielsweise Alcofenac, Aceclofenac, Sulindac, Tolmetin, Etodolac, Fenopren, Thiaprofensäure, Meclofenamsäure, Meloxicam, Tenoxicam, Lornoxicam, Nabumeton, Acetaminophen, Phenacetin, Ethenzamid, Sulpyrin, Mefanamsäure, Flufenamsäure, Diclofenacnatrium, Loxoprofennatrium, Phenylbutazon, Indomethacin, Ibuprofen, Ketoprofen, Naproxen, Oxaprozin, Flurbiprofen, Fenbufen, Pranoprofen, Floctafenin, Piroxicam, Epirizol, Tiaramidhydrochlorid, Zaltoprofen, Gabexatmesylat, Camostatmesylat, Ulinastatin, Colchicin, Probenecid, Sulfinpyrazon, Benzbromaron, Allopurinol, Salicyläure, Atropin, Scopolamin, Levorphanol, Ketorolac, Tebufelon, Tenidap, Clofezon, Oxyphenbutazon, Prexazon, Apazon, Benzydamin, Bucolom, Cinchopen, Clonixin, Ditrazol, Epirizol, Fenoprofen, Floctafeninl, Glaphenin, Indoprofen, Nifluminsäure, Suprofen, Bufexamac, Dexamethason, Hexestrol, Methimazol, Betamethason, Triamcinolon, Fluocinonid, Prednisolon, Methylprednisolon, Hydrocortison, Fluorometholon, Beclomethasondipropionat, Estriol, Clobetasol, Diflorasondiacetat, Halbetosalpropionat, Amicinonid, Desoximetason, Halcinonid, Mometasonfuroat, Fluticasonpropionat, Flurandrenolid, Clocortalon, Predincarbat, Aclometasondipropionat oder Desonid.

Auch Vitamine wie beispielsweise Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure können der Zusammensetzung zugegeben werden.

Flüssige Formulierungen umfassen Lösungen, Suspensionen, Sprays und Emulsionen.

Kapseln werden beispielsweise aus Methylcellulose, Polyvinylalkohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragant und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Zucker, natürliche als auch synthetische Gummis wie beispielsweise Akaziengummi oder Guar-Gummi, Gelatine, Tragant, Alginsäure, Natriumalginat, Ammonium-Calcium-Alginat, Methylcellulose, Carboxymethyl-Cellulose, Polyethylenglycol, Wachse, Natrium-Carboxymethylcellulose, Hydroxypropylmethylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Insbesondere bevorzugt sind Mikroemulsionen und Nanoemulsionen, welche als Emulsion, Salbe, Creme oder auch Gel formuliert werden können.

Als "Mikroemulsion" wie hierin verwendet wird eine Öl in Wasser Emulsion bezeichnet, wobei der Extrakt aus *Pandanus conoideus* sich vorzugsweise in der Ölphase befindet und die Öltröpfchen einen durchschnittlichen Durchmesser von 1 µm - 50 µm aufweisen. Die Mikroemulsionen können nach bekannten Verfahren und mit bekannten Zusatzstoffen wie auch in der vorliegenden Anmeldung beschrieben hergestellt werden. Derartige Formulierungen sind für die topische Anwendung überraschend besser geeignet als Emulsionen mit größeren Tröpfchendurchmesser von größer als 200 µm oder von Emulsionen mit Tröpfchendurchmesser von 200 µm - 400 µm. Die Tröpfchengröße wird durch die Art und Stärke der Vermischung von Ölphase in Wasserphase eingestellt.

Somit sind Mikroemulsionen mit einem durchschnittlichen Durchmesser der Öltropfen in der Mikroemulsion von 1 µm - 50 µm, bevorzugt von 5 µm - 40 µm und weiter bevorzugt von 10 µm - 30 µm besonders gut zur Behandlung von Erkrankungen der Haut und der Schleimhaut im Bereich von Nase, Augen und Ohren sowie insbesondere zur Behandlung und Prophylaxe von trockener Haut, Neurodermitis, Dermatitis, Ekzemen, Psoriasis, Stoffwechselstörungen der Haut, Akne, geschädigter Haut und Alterungserscheinungen sowie zur Glättung der Haut und zur Steigerung der Feuchtigkeitsversorgung der Haut geeignet.

Als "Nanoemulsion" wie hierin verwendet wird eine Öl in Wasser Emulsion bezeichnet, wobei der Extrakt aus *Pandanus conoideus* sich vorzugsweise in der Ölphase befindet und die Öltröpfchen einen durchschnittlichen Durchmesser von 50 nm - 800 nm aufweisen. Die Nanoemulsionen können nach bekannten Verfahren und mit bekannten Zusatzstoffen wie auch in der vorliegenden Anmeldung beschrieben hergestellt werden. Derartige Formulierungen sind für die topische Anwendung überraschend besser geeignet als Emulsionen mit größeren Tröpfchendurchmesser von größer als 200 µm.

Somit sind Nanoemulsionen mit einem durchschnittlichen Durchmesser der Öltropfen in der Mikroemulsion von 50 nm - 800 nm, bevorzugt von 100 nm - 600 nm und weiter bevorzugt von 200 nm - 400 nm besonders gut zur Behandlung von Erkrankungen der Haut und der Schleimhaut im Bereich von Nase, Augen und Ohren sowie insbesondere zur Behandlung und Prophylaxe von trockener Haut, Neurodermitis, Dermatitis, Ekzemen, Psoriasis, Stoffwechselstörungen der Haut, Akne, geschädigter Haut und Alterungserscheinungen sowie zur Glättung der Haut und zur Steigerung der Feuchtigkeitsversorgung der Haut geeignet.

Tröpfchengrößen unter 1 µm wurden basierend auf ihren Zetapotentialen mittels Nicomp-Gerät von Particle Sizing System Inc. bestimmt. Für größere Tröpfchen (1 µm - 200 µm) wurden optische Einzelpartikel-Analysen mittels PSS-Accusizer-Gerät durchgeführt.

Diese bevorzugte Eignung von Mikroemulsionen und Nanoemulsion mit durchschnittlichen Tröpfchengrößen von 50 nm bis 50 µm insbesondere zur Behandlung und Prophylaxe von trockener Haut, Neurodermitis, Dermatitis, Ekzemen, Psoriasis, Stoffwechselstörungen der Haut, Akne, geschädigter Haut und Alterungserscheinungen der Haut ist daher überraschend, weil offenbar solche Formulierungen geeignet sind, den Pandanusextrakt effektiv an den Wirkort, d.h. wo er benötigt wird, zu bringen. Dies zeigt sich besonders eindrucksvoll an der unerwartet hohen klinischen Heilungsrate in der Anwendungsstudie (Beispiel 11: Reduktion SCORAD Index von ca. 79 % nach 3 Wochenbehandlung). Solch hohe Heilungsraten sind mit der Wirksamkeit der Hydrocortison-Therapie oder auch mit der Wirksamkeit von stärkeren Cortisonpräparaten vergleichbar und daher für einen Pflanzenextrakt aus unzähligen Komponenten sehr verwunderlich und für einen Fachmann nicht zu erwarten. Es ist daher durchaus für einen Fachmann überraschend, dass ein mittels einfacher Extraktionsverfahren gewonnener Pandanusextrakt ohne weitere aufwendige Reinigungsverfahren wie z.B. Chromatographie und ohne weitere Isolierung bestimmter Wirkstoffe aus dem Extrakt eine derart starke therapeutische Wirkung zeigt, welche man ansonsten nur von reinen Wirkstoffen wie z.B. dem Cortison her kennt. Bevorzugt sind somit Formulierungen als Creme, Salbe, Gel oder Emulsion, welche durchschnittliche Tröpfchendurchmesser von kleiner als 50 µm, bevorzugt kleiner als 1 µm und insbesondere bevorzugt kleiner als 500 nm aufweisen. Die Bevorzugung solcher Mikroformulierungen und Nanoformulierungen ergibt sich auch aus den Beispielen 11 bis 14.

### Figurenbeschreibung:

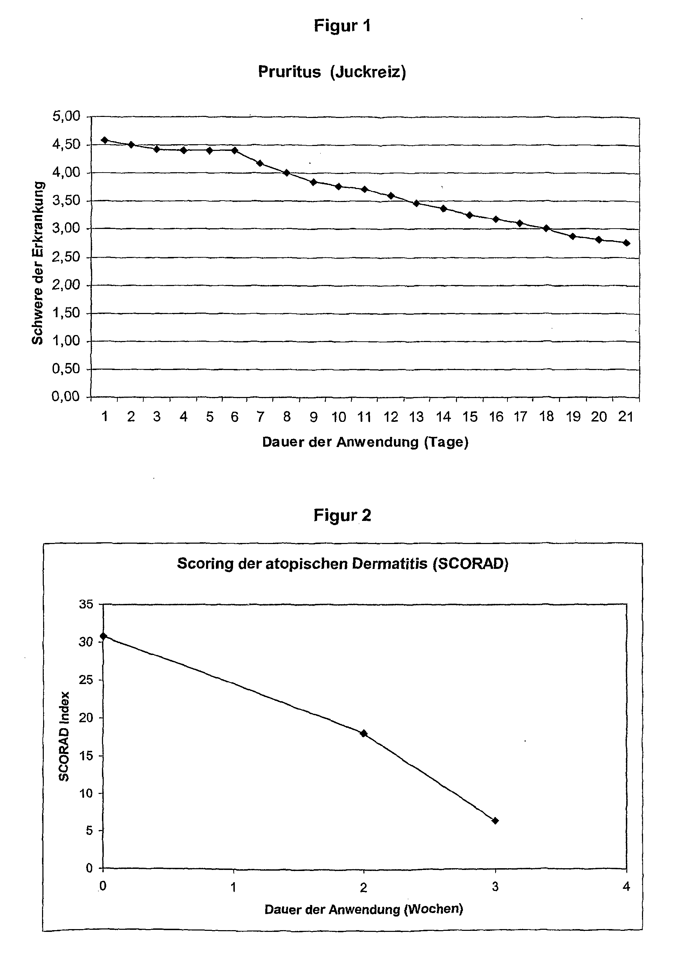

- Figur 1: zeigt einen deutlichen Rückgang des Juckreizes bei Neurodermitis-Patienten bereits nach 21 Tagen bei Anwendung des erfindungsgemäßen Extraktes so wie in Beispiel 11 definiert.
- Figur 2: zeigt den Krankheitsverlauf der Neurodermitis gemäß dem SCORAD Index nach 3-wöchiger Anwendung des erfindungsgemäßen Extraktes so wie in Beispiel 11 definiert.
- Figur 3: zeigt den Effekt von Pandanus-Extrakt auf die LPS induzierte TNF-α Ausschüttung in humanen Monozyten
- Figur 4: zeigt den Effekt von Pandanus-Extrakt auf die LPS induzierte IL-6 Ausschüttung in humanen Monozyten
- Figur 5: zeigt den Effekt von Pandanus-Extrakt auf die LPS induzierte PGE-2 Ausschüttung in humanen Monozyten

### Beispiele

### Beispiel 1:

### Gewinnung von kaltgepreßtem Öl

Die rote, kernhaltige Fruchtschicht wird vorzugsweise mit Zusatz von Wasser in einem Mixer gerührt um das rote Fruchtfleisch von den Kernen zu trennen. Das gewonnene, rote Fruchtfleischbrei werden gepresst. Nach Zentrifugation und Filtration gewinnt man ein klares, rotes, dickflüssiges Öl (kalt gepresstes Virgin-Öl; Extrakt 1). Die Kerne werden mit Wasser gewaschen und bei einer Temperatur von höchstens 40°C im Trockenschrank getrocknet und für die weitere Verarbeitung aufbewahrt.

### Beispiel 2:

### Gewinnung des Öls durch eine Extraktion mit organischen Lösungsmitteln

Das wie oben gewonnene, rote Fruchtfleischbrei wird mit einem lipophilen Lösungsmittel, z.B. Hexan, Pentan, Cyclohexan, Petrolether, Heptan, tert-Butylmethylether extrahiert.
Nach Trocknung (Wasserentzug), Zentrifugation und Filtration erhält man das rote, dickflüssige Öl.

### Beispiel 3:

### Gewinnung der Alkoholextrakte

Der wie oben gewonnene, rote Fruchtfleischbrei wird mit einem polaren Lösungsmittel wie beispielsweise einem Alkohol, Ether, Aceton, THF, Essigsäureethylester und bevorzugt Methanol oder Ethanol extrahiert.
Nach Abdampfen des Lösungsmittels erhält man den alkoholischen Extrakt.

### Beispiel 4:

### Gewinnung der Alkoholextrakte aus den Kernen

Die wie oben beschrieben gewonnenen Kerne werden zerkleinert und mit 50%igem Methanol-Wasser extrahiert. Nach Abdampfen des Lösungsmittels erhält man den Extrakt der Kerne (Kernextrakt).

### Beispiel 5:

### Gewinnung des Extraktes aus der getrockneten roten Fruchtschicht samt den Kernen durch eine Extraktion mit organischen Lösungsmitteln

Der rote Fruchtanteil samt den Kernen wird bei unter 40 °C im Trockenschrank getrocknet. Das Wassergehalt des getrockneten Material soll unter 12% sein. Das trockene Material wird zerkleinert und mit organischen Lösungsmittel wie Methanol, Ethanol, Aceton, Isopropylalkohol oder Hexan, vorzugsweise mit Ethanol, extrahiert. Man erhält ein dunkel-rotes, dickflüssiges öliges Produkt (Oleosum).

Solche Extraktion kann selbstverständlich auch mit frischem Fruchtmaterial durchgeführt werden.

### Beispiel 6:

### Gewinnung des wässrigen Saftes aus der weißen/hellen, inneren Fruchtschicht

Aus der weißen Schicht, d.h. dem inneren hellen Fruchtfleischanteil wird durch direkte Verpressung eine wässrige Phase gewonnen. Stabilisatoren wie z.B. Ascorbinsäure oder andere Antioxidantien können dem Saft zugefügt werden, um Oxidation durch Luftsauerstoff zu verhindern. Der Saft kann direkt eingesetzt oder für spätere Anwendungen getrocknet werden.

### Beispiel 7:

### Gewinnung der Alkoholextrakte aus dem inneren, hellen Fruchtanteil

Der weiße, helle Fruchtanteil wird in Scheiben von ca. 4x4x2 cm geschnitten und im Trockenschrank getrocknet. Die Stücke werden zerkleinert und mit 50% Methanol-Wasser oder 50% Ethanol-Wasser extrahiert.

### Beispiel 8:

### Extraktgewinnung aus dem roten Fruchtfleisch mit überkritischem Kohlendioxid (Tk = 31°C, Pk = 73,9 bar)

Im Extraktionsbehälter wird das rote Fruchtbrei mit dem flüssigen Kohlendioxid versetzt ( 90 bar, 8 kg CO₂/h über 4 h). Das mit Pflanzeninhaltsstoffen beladene Lösungsmittel wird mit Hilfe einer Pumpe in den Abscheidebehälter gepumpt. Auf dem Weg dorthin wird der Druck mittels eines Drosselventils auf unterkritische Bedingungen entspannt. Die Löslichkeit nimmt ab, und die gelösten lipophilen Stoffe fallen aus. Im Abscheidebehälter werden die lipophilen Stoffe (Ölfraktion) vom Gas getrennt. Das Gas wird zum Fluid rekomprimiert und wieder verwendet. Durch Zugabe verschiedener Konzentrationen von Schleppmitteln (z.B. Methanol oder Ethanol) können auch mittelpolare und auch polare Substanzen schonend extrahiert werden.

### Beispiel 9:

### Zubereitung der Extrakte

Nach dem gründlichen Waschen der Frucht mit Wasser wird die äußere rote Schicht von der inneren weißen Schicht getrennt. Aus der roten Schicht wird durch Schütteln und/oder Reiben der Fruchtfleisch-Anteil von den Kernen getrennt.

Der rote Fruchtfleisch-Anteil wird homogenisiert, gepresst und zentrifugiert. Nach der Zentrifugation wird die Ölphase abgetrennt (Extrakt 1). Der Rückstand wird zuerst mit n-Hexan (Extrakt 2) und danach mit Methanol (Extrakt 3) extrahiert.

Die Kerne werden zerkleinert und mit 50% Methanol extrahiert (Extrakt 4).

Aus der weißen Schicht wird durch die direkte Verpressung eine wässrige Phase (Extrakt 5) gewonnen. Der Rückstand nach der Verpressung wird getrocknet und mit 50%igem Methanol extrahiert (Extrakt 6).
Alle Arbeitsvorgänge werden unter 40°C durchgeführt.

### Beispiel 10:

### Herstellung topischer Formulierungen

Soweit nicht anders angegeben, handelt es sich bei den Mengenangaben um Gewichtsprozent auf das Gewicht und die Gesamtmenge bzw. auf das Gesamtgewicht der Zubereitung. Die beschriebenen Formulierungen sind als kosmetische und auch als pharmazeutische Formulierungen geeignet. Bei den pharmazeutischen Formulierungen ist der Anteil an Pandanusextrakt in der Regel höher vorzugsweise doppelt so hoch wie bei den kosmetischen Formulierungen.
Formulierung 1:

| Nr. | Bestandteile | Anteil (Gew.-%) |
|---|---|---|
| 1 | Lecithin | 4,0 |
| 2 | Extrakt gemäß Beispiel 5 | 1,0 |
| 3 | Glycerin | 5,0 |
| 4 | Propylenglykol | 16,0 |
| 5 | Mittelkettige Triglyceride | 3,0 |
| 6 | Wollwachs | 10,0 |
| 7 | gereinigtes Wasser | ad 100 |

Formulierung 2:

| Nr. | Bestandteile | Anteil (Gew.-%) |
|---|---|---|
| 1 | Extrakt gemäß Beispiel 8 | 2,0 |
| 2 | Zinkoxid | 10,0 |
| 3 | Glycerin | 6,0 |
| 4 | Dickflüssiges Paraffin | 30,0 |
| 5 | Weißes Vaselin | 20,0 |
| 6 | Gebleichtes Wachs | ad 100 |

Formulierung 3:

| Nr. | Bestandteile | Anteil (Gew.-%) |
|---|---|---|
| 1 | Extrakt gemäß Beispiel 2 | 2,0 |
| 2 | Dexpanthenol | 1,5 |
| 3 | Miglyol | 5,0 |
| 4 | gereinigtes Wasser | 5,0 |
| 5 | Basiscreme DAC | Ad 100 |

Formulierung 4:

| Nr. | Bestandteile | Anteil (Gew.-%) |
|---|---|---|
| 1 | Extrakt gemäß Beispiel 3 | 3,0 |
| 2 | Mittelkettige Triglyceride | 5,0 |
| 3 | Polysorbat 60 | 8,0 |
| 4 | Propylenglycol | 6,0 |
| 5 | Erdnussöl | 10,0 |
| 6 | Glycerin | 10,0 |
| 7 | Vaselinum album | ad 100 |

Formulierung 5:

| Nr. | Bestandteile | Anteil (Gew.-%) |
|---|---|---|
| 1 | Extrakt gemäß Beispiel 4 | 8,0 |
| 2 | Harnstoff | 4,0 |
| 3 | Milchsäure | 1,0 |
| 4 | Kaliumsorbat | 0,15 |
| 5 | gereinigtes Wasser | 35,0 |
| 6 | Wollwachsalkoholsalbe | ad 100 |

Formulierung 6:

| Nr. | Bestandteile | Anteil (Gew.-%) |
|---|---|---|
| 1 | Extrakt gemäß Beispiel 7 | 6,0 |
| 2 | Mittelkettige Triglyceride | 4,0 |
| 3 | Basiscreme DAC | ad 100 |

Formulierung 7:

| Nr. | Bestandteile | Anteil (Gew.-%) |
|---|---|---|
| 1 | Extrakt 1 und Extrakt 4 (80 : 20) | 4,0 |
| 2 | Unguentum emulsificans aquosum | 45,0 |
| 3 | gereinigtes Wasser | Ad 100 |

Formulierung 8:

| Nr. | Bestandteile | Anteil (Gew.-%) |
|---|---|---|
| 1 | Extrakt 2 und Extrakt 5 (40 : 60) | 3,0 |
| 2 | Zinkoxid | 15,0 |
| 3 | Talkum | 15,0 |
| 4 | Glycerol | 30,0 |
| 5 | Propylenglycol | 10,0 |
| 6 | gereinigtes Wasser | ad 100 |

Formulierung (O/W) 9:

| Nr. | Bestandteile | Anteil (Gew.-%) |
|---|---|---|
| 1 | Extrakt gemäß Beispiel 3 | 2,0 |
| 2 | Glycerylsteratcitrat | 1,0 |
| 3 | Polyethylenglycal(20)cetearylether | 10,0 |
| 4 | Dimethicon | 0,5 |
| 5 | Behenylalkohol | 1,0 |
| 6 | Dicaprylylcarbonat | 3,0 |
| 7 | Tocopherol | 0,5 |
| 8 | Octyldodecanol | 0,5 |
| 9 | Panthenol | 0,5 |
| 10 | Carbomer | 0,05 |
| 11 | Caprylic Triglycerid | 1,0 |
| 12 | Methylparaben | 0,4 |
| 13 | Propylparaben | 0,3 |
| 14 | Sorbitol | 10,0 |
| 15 | Wasser | ad 100 |

Formulierung (Gel) 10:

| Nr. | Bestandteile | Anteil (Gew.-%) |
|---|---|---|
| 1 | Extrakt gemäß Beispiel 5 | 4,0 |
| 2 | Acrylat/C10-30 Alkylacrylat Crosspolymer | 0,2 |
| 3 | Polyacrylsäure | 0,10 |
| 4 | Xanthan Gummi | 3,0 |
| 5 | Cetearylalkohol | 4,0 |
| 6 | C12-15 Alkylbenzoat | 3,0 |
| 7 | Caprylic/Capric Triglycerid | 5,0 |
| 8 | Zyklischer Dimethylpolysiloxan | 1,0 |
| 9 | Isopropanol | 3,0 |
| 10 | Natriumhydroxid | q.s. |
| 11 | Wasser | ad 100 |

Formulierung (Wasserfrei Formulierung) 11:

| Nr. | Bestandteile | Anteil (Gew.-%) |
|---|---|---|
| 1 | Extrakt gemäß Beispiel 8 | 4,0 |
| 2 | Polyglyceryl-3-Diisostearate | 3,5 |
| 3 | Glycerin | 14,0 |
| 4 | Polyglyceryl-2-Dipolyhydroxystearate | 3,5 |
| 5 | Miglyol | 6,0 |
| 6 | Propylenglykol | 2,0 |
| 7 | Sonnenblumenöl | 4,0 |
| 8 | Vaselinum album | ad 100 |

Formulierung (O/W) 12:

| Nr. | Bestandteile | Anteil (Gew.-%) |
|---|---|---|
| 1 | Extrakt gemäß Beispiel 1 +4 (80:20) | 6,0 |
| 2 | Glycerylstearat | 3,0 |
| 3 | PEG-100-Stearat | 0,75 |
| 4 | Behenylalkohol | 2,0 |
| 5 | Caprylic-/Capric-Triglycerid | 8,0 |
| 6 | Octyldodecanol | 5,0 |
| 7 | C12-15 Alkylbenzoat | 3,0 |
| 8 | Magnesiumsulfat | 0,8 |
| 9 | Ethylendiamintetraessigsäure | 0,1 |
| 10 | Wasser | ad 100. |

Formulierung (O/W) 13:

| Nr. | Bestandteile | Anteil (Gew.-%) |
|---|---|---|
| 1 | Extrakt gemäß Beispiel 4 | 4,0 |
| 2 | Glycerylstearat | 2,0 |
| 3 | Natriumcitrat | 0,174 |
| 4 | Zitronensäure | 0,086 |
| 5 | Glycerin | 10,0 |
| 6 | 1,3 Butylenglykol | 0,5 |
| 7 | Hexandiol | 0,5 |
| 8 | Phenoxyethanol | 0,25 |
| 9 | Benzylalkohol | 0,2 |
| 10 | Ethylparaben | 0,1 |
| 11 | Propylparaben | 0,1 |
| 12 | Phospholipon 90 G | 8,0 |
| 13 | Silikonöl, zyklisch | 3,0 |
| 14 | Pentaerythrityltetraisostearin | 3,0 |
| 15 | Triisostearin | 3,0 |
| 16 | BHT | 0,05 |
| 17 | PEG-40 Stearat | 1,0 |
| 18 | Cetylalkohol | 2,0 |
| 19 | Caprylic/Capric Triglycerid | 3,0 |
| 20 | Wasser | ad 100 |

Formulierung (Öl) 14:

| Nr. | Bestandteile | Anteil (Gew.-%) |
|---|---|---|
| 1 | Extrakt gemäß Beispiel 1 | 3,0 |
| 2 | Jojobaöl | 5,0 |
| 3 | Kakaobutter | 0,05 |
| 4 | Macadamianußöl | 5,0 |
| 5 | Medizinisches Weißöl | 30,0 |
| 6 | Caprylic/Capric Triglycerid | 5,0 |
| 7 | Rizinusöl | 5,0 |
| 8 | Ethylhexylstearat | 10,0 |
| 9 | Tocopherolacetat | 0,1 |
| 10 | Tocopherol | 0,05 |
| 11 | Sonnenblumöl | ad 100 |

Formulierung (W/O) 15:

| Nr. | Bestandteile | Anteil (Gew.-%) |
|---|---|---|
| 1 | Extrakt gemäß Beispiel 6 | 2,0 |
| 2 | PEG-30 Dipolyhydroxystearat | 0,5 |
| 3 | Lanolin Alcohol | 1,5 |
| 4 | Paraffinöl | 10,0 |
| 5 | Vaselin | 6,0 |
| 6 | Hydrierte Kokosglyceride | 1,0 |
| 7 | Hydriertes Polyisobuten | 0,75 |
| 8 | Octyldodecanol | 1,0 |
| 9 | Aluminiumstearat | 0,3 |
| 10 | Dicaprylylcarbonat | 0,05 |
| 11 | Hydriertes Rizinusöl | 7,5 |
| 12 | Magnesiumsulfat | 0,6 |
| 13 | Tetraatriumiminosuccinat | 0,6 |
| 14 | Capryl-/Caprinsäuretriglycerid | 2,5 |
| 15 | Methylparaben | 0,15 |
| 16 | Propylparaben | 0,4 |
| 17 | Glycerin | 5,0 |
| 18 | Wasser | ad 100 |

Formulierung (Creme) 16:

| Nr. | Bestandteile | Anteil (Gew.-%) |
|---|---|---|
| 1 | Extrakt gemäß Beispiel 7 | 5,0 |
| 2 | Glycerylstearat | 2,5 |
| 3 | Stearylalkohol | 2,0 |
| 4 | PEG-40-Stearat | 1,0 |
| 5 | Cetylalkohol | 2,0 |
| 6 | SHEABUTTER | 2,0 |
| 7 | Carrageen | 3,0 |
| 8 | Tapiokastärke | 1,0 |
| 9 | Ethylhexylmethoxycinnamat | 2,0 |
| 10 | Phenoxyethanol | 0,4 |
| 11 | C₁₂₋₁₅Alkylbenzoat | 3,0 |
| 12 | p-Hydroxybenzoesäurealkylester (Paraben) | 0,4 |
| 13 | Aristoflex AVC (Ammonium Polyacryldimethyltaurid / Vinylformamid-copolymer) | 0,2 |
| 14 | 2-Isopropyl-5-Methyl-Cyclohexancarbonyl-D-Alaninethylexter | 0,5 |
| 15 | Glycerin | 8,0 |
| 16 | EDTA | 0,2 |
| 17 | Wasser | ad 100 |

### Beispiel 11: klinische Untersuchungen

### PRINZIP UND METHODIK:

Ziel dieser Untersuchung war es, den Extrakt exakt auf seine Verträglichkeit und Wirksamkeit nach klinisch-dermatologischen Prüfkriterien zu untersuchen. Die Patienten konnten täglich den testbegleitenden Dermatologen bei objektiven und subjektiven Hautveränderungen zu Rate ziehen.
Zu Beginn der Studie, nach 2 Wochen Anwendung sowie nach 3 Wochen Anwendung, erfolgten die dermatologischen Untersuchungen der Studienteilnehmer mit Erhebung des modifizierten SCORAD's.
Jede Testperson wandte anschließend den Extrakt zweimal täglich insgesamt drei Wochen lang auf den betroffenen Hautstellen an. Bei Bedarf konnten tägliche Kontrollen des Hautzustandes durch den testbegleitenden Dermatologen durchgeführt werden.

### Der verwendete Extrakt:

Der in der klinischen Studie eingesetzte Extrakt wurde gemäß Beispiel 5 hergestellt und als Creme mit einer Konzentration von 3% Pandanusextrakt formuliert.

**Tab. 1 Zusammensetzung der angewendeten Nanoemulsion-Creme für die Anwendungsstudie bei Neurodermitikern**

| | % |
|---|---|
| Dipalmitoyl-Phosphatidylcholin | 5,000 |
| Glycerol 85% | 1,500 |
| Dexpanthenol | 1,000 |
| Urea | 3,000 |
| Pandanusextrakt (Beispiel 5, Ethanolextrakt) | 3,000 |
| Tocopherolacetat | 0,200 |
| Sodium benzoate | 0,150 |
| Aq. Dest. | 86,15 |
| | 100,00 |

### Patienten:

Das Patientenkollektiv bestand aus erwachsenen Testpersonen mit Hautveränderungen der Neurodermitis, i.S. Juckreiz, Rötung, Schuppung oder Trockenheit. Untersucht wurden 20 Personen mit leichter bis mittelstarker Neurodermitis.

| **Nr.** | **Geschlec ht** | **Alter** | **Diagnose** | **Applikationsort** |
|---|---|---|---|---|
| **1.** | w | 65 | Neurodermitis | Unterschenkel |
| **2.** | w | 52 | Neurodermitis | Oberarm |
| **3.** | w | 57 | Neurodermitis | Oberkörper |
| **4.** | w | 52 | Neurodermitis | Oberkörper |
| **5.** | w | 30 | Neurodermitis | Ellenbeuge |
| **6.** | w | 36 | Neurodermitis | Oberkörper |
| **7.** | w | 44 | Neurodermitis | Oberkörper |
| **8.** | m | 45 | Neurodermitis | Bein |
| **9.** | m | 42 | Neurodermitis | Unterarme |
| **10.** | w | 67 | Neurodermitis | Kniekehle, Fußsohle |
| **11.** | w | 40 | Neurodermitis | Oberkörper |
| **12.** | w | 55 | Neurodermitis | Ellenbeuge |
| **13.** | m | 45 | Neurodermitis | Oberkörper |
| **14.** | m | 48 | Neurodermitis | Ellenbeuge |
| **15.** | w | 73 | Neurodermitis | Kniekehle |
| **16.** | m | 37 | Neurodermitis | Unterarme |
| **17.** | w | 31 | Neurodermitis | Kniekehle |
| **18.** | m | 53 | Neurodermitis | Ellenbeuge |
| **19.** | w | 70 | Neurodermitis | Oberarme |
| **20.** | w | 68 | Neurodermitis | Unterschenkel |

### Ausschlusskriterien:

- Akute organische Krankheit
- Schwangerschaft und Stillzeit
- Bestehende Sensibilisierung auf Inhaltsstoffe der Prüfprodukte
- Schwerwiegende Erkrankungen
- Applikation von wirkstoffhaltigen Präparaten und Pflegemitteln bis 4 Wochen vor Testbeginn
- Einnahme von Medikamenten gegen Neurodermitis/ Atopie (Glucocorticoide, Antiallergika, topische Immunmodulatoren, etc.)

### Durchführung:

Es wurden nur Teilnehmerinnen und Teilnehmer in die Prüfgruppe aufgenommen, bei denen sich keinerlei sonstige behandlungsbedürftige Hautveränderungen fanden.
Zu Beginn der Studie, nach 2 Wochen Anwendung sowie nach 3 Wochen Anwendung, erfolgte eine dermatologische Untersuchung der Studienteilnehmer mit Erhebung des ärztlichen Beurteilungsbogens.
Der Extrakt wurde den Probanden mit der Applikationsinstruktion über mindestens 2x-täglicher Applikation ausgehändigt. Weiterhin wurden die Probanden instruiert, in der Zeit des Anwendungstests auf andere Präparate zu verzichten.

### Atopie-Score (modifiziert nach SCORAD):

Dieser Index dient der qualitativen und quantitativen Einschätzung des Schweregrades des atopischen Ekzems, modifizierter SCORAD (= Severity Scoring of Atopic Dermatitis).
Die standardisierte Beurteilung erfolgt durch die Angabe :
A) des Ausprägungsgrades sechs typischer morphologischer Veränderungen (Grad 0-3, Gesamtwert hier: max 10)
B) des Anteils der betroffenen Hautfläche (%) bzgl. Rötung / Kruste / Excoriation
C) der subjektiven Einschätzung von Juckreiz anhand einer visuellen Analogscala (Grad 0-10; hier: max. 7), außerdem des nächtlichen Schlafverlustes (Grad 0-10, hier: max 3).

### zu A)

Klassifiziert werden sechs typische morphologische Veränderungen:
Hautrötung
Krusten
flächenhafte Infiltration d. Haut mit Vergröberungen der Hautfelder
Schwellung/Bläschen (hier: Ausschlußkriterium)
Hautabschürfung bis ins Korium (hier: Ausschlußkriterium)
Trockenheit (wird an den NICHT betroffenen Hautstellen erhoben).
Kriterium: nicht vorhanden = 0 / milde vorhanden = 1 / mittelgradig = 2 / ausgeprägt = 3

Die Summe der Werte kann zwischen 0-10 schwanken, wobei die Ausprägung von Ödem oder Papeln als Ausschlußkriterium definiert wurde, als Anhalt für einen akuten neurodermitischen Hautzustand mit medizinischer Behandlungsbedürftigkeit. Hautabschürfung bis ins Korium wurde als Ausschlußkriterium im Rahmen der Eingangsuntersuchung definiert. Exkoriationen im Verlauf der Studie waren Indikation für eine Testunterbrechung von 2 Tagen.

### Zu B)

Überschreitet der Gesamtwert des SCORAD 50 aufgrund einer großflächigen Ausprägung führt dies ebenfalls zum Ausschluß des Probanden.

### Zu C)

Die Einschätzung des Juckreizes >7 und des Schlafverlustes >3 führt ebenfalls zum Ausschluß, da medizinische Behandlungsbedürftigkeit, evtl. Artefakte anzunehmen sind.

Der maximale Gesamtwert als modifizierter SCORAD nach Dermatest ist 50, als Ausdruck einer mittleren Ausprägung des atopischen Krankheitsbildes. Berechnung des Index: 7A/2+B/5+C.

### Dermatologische Untersuchungen:

### 1. Vor Beginn des Anwendungstests

Alle 20 Studienteilnehmer zeigten entweder leichte Rötung, Schuppung oder Trockenheit. Weitere pathologische Hautveränderungen waren in keiner Form festzustellen.

### 2. Während des Anwendungstests

Keine der 20 Studienteilnehmer zeigte im Verlauf des dreiwöchigen Anwendungstests pathologische Hautveränderungen. Testunterbrechungen oder gar hautfachärztliche Behandlungen waren nicht notwendig.

### 3. Nach Ende des Anwendungstests

Bei der dermatologischen Abschlussuntersuchung nach dem Testende zeigten sich bei den 20 Studienteilnehmern keine zusätzlichen pathologische Hautveränderungen. Der Extrakt wurde sehr gut vertragen und führte bei keiner Testperson zu unerwünschten Hautveränderungen.

### Dermatologische Beurteilungskriterien erfaßt durch mod. SCORAD:

| | |
|---|---|
| 1. Ödem/Papeln | 5. Lichenifikation |
| 2. Rötung/Erythem | 6. Trockenheit (Xerosis) |
| 3. Krustenbildung | 7. Verstärkter Juckreiz |
| 4. Excoriation | 8. Schlafverlust |

### Subjektive Beurteilungskriterien:

| | |
|---|---|
| 1. | Schweregrad der Erkrankung |
| 2. | Rötung/Erythem |
| 3. | Trockenheit |
| 4. | Juckreiz |
| 5. | Wirkung des Prüfpräparates |

Die Bewertung erfolgt durch die Probanden im Rahmen der täglichen Dokumentation im Probandentagebuch. Die Skala umfaßt den Bereich 0-7, 0 = nicht vorhanden, 7= sehr stark vorhanden.

### BEURTEILUNG DER TESTERGEBNISSE:

Ingesamt 20 Studienteilnehmer vertrugen im insgesamt dreiwöchigen Anwendungstest nach dermatologisch-klinischen Kriterien den Extrakt einwandfrei. Es kam in keinem Fall zu unerwünschten oder gar pathologischen Hautveränderungen. Nach der Anwendung des Extraktes über 14 Tage ergab sich eine Reduktion des durchschnittlichen SCORAD-Wertes von 30,79 auf 18,04. Dies entspricht einer Verminderung um -41,40 % zum Ausgangswert.

Nach der erfindungsgemäßen Anwendung des Extraktes über 21 Tage ergab sich bei den Studienteilnehmern eine Reduktion des durchschnittlichen SCORAD-Wertes von 30,79 auf 6,4. Dies entspricht einer Verminderung der Symptome um -79,23 % zum Ausgangswert.

Der Rückgang des Atopie-Index ist daher als exzellent zu bewerten.

### Beispiel 12: Erfahrungsbericht mit Pandanus-Extrakt-Microemulsionen ohne hautpflegende Zusatzstoffe

Pandanus-Extrakt-Microemulsion ohne weitere Zugabe der hautpflegenden Stoffe (Zusammensetzung siehe Tab. 2)

Die Anwendung von Pandanus-Extrakt-Microemulsion wurde bei drei Probanden mit atopischem Ekzem und mit symmetrischen befallenen Stellen beobachtet. Die befallene Stelle wurde entweder mit Pflege-Lotio (freie Wahl) oder mit Pandanus-Microemulsion zweimal täglich behandelt (links-rechts-Vergleich). Die Behandlungs- und Beobachtungszeit war eine Woche. Die Veränderung der Hauptsymptomatik der Erkrankung, nämlich Juckreiz, wurde vor und nach der Behandlung und zwischen den Behandlungen verglichen. Alle 3 Probanden hatten vor Beginn der Behandlung starke bis mittelstarke Juckreiz-Beschwerden. (Abstufung der Juckreiz-Symptomatik: starke - mittelstarke - milde - keine Beschwerden)

**Tab.2 Zusammensetzung der Microemulsion nur mit Pandanusextrakt und Emulgatoren**

| | % |
|---|---|
| Lipoid S 20 | 12,00 |
| Mygliol | 4,00 |
| Ethanolischer Pandanusextrakt (Beispiel 5) | 8,00 |
| Sodium benzoate | 0,15 |
| Aq. dest. | 80,85 |
| | 100,00 |

Nach 1 Woche Behandlung mit der Pandanus-Microemulsion berichteten die Probanden eine starke Abnahme des Juckreizes (milde Beschwerden : 2 / keine Beschwerden : 1 Proband), wohingegen die Anwendung mit Pflege-Lotio weniger Erleichterung im Vergleich zu der Anwendung mit Pandanus-Extrakt zeigte (starke Beschwerden 1 / mittelstarke Beschwerden 2 Probanden).
Pandanus-Extrakt-Nanoemulsion ohne weitere Zugabe der hautpflegenden Stoffe (Zusammensetzung siehe Tab. 2)

### Beispiel 13: Erfahrungsbericht mit Pandanus-Extrakt-Nanoemulsionen ohne hautpflegende Zusatzstoffe

Die Anwendung von Pandanus-Extrakt-Nanoemulsion wurde bei drei Probanden mit atopischem Ekzem und mit symmetrischen befallenen Stellen beobachtet. Die befallene Stelle wurde entweder mit Pflege-Lotio (freie Wahl) oder mit Pandanus-Nanoemulsion zweimal täglich behandelt (links-rechts-Vergleich). Die Behandlungs- und Beobachtungszeit war eine Woche. Die Veränderung der Hauptsymptomatik der Erkrankung, nämlich Juckreiz. wurde vor und nach der Behandlung und zwischen den Behandlungen verglichen. Alle 3 Probanden hatten vor Beginn der Behandlung ein starkes bis mittelstarkes Juckreiz-Leiden. (Abstufung der Juckreiz-Symptomatik: starke - mittelstarke - milde - keine Beschwerden)

**Tab.3 Zusammensetzung der Nanoemulsion nur mit Pandanusextrakt und Emulgatoren**

| | % |
|---|---|
| Dipalmitoyl-Phosphatidylcholin | 6,00 |
| Mygliol | 8,00 |
| Ethanolischer Pandanusextrakt (Beispiel 5) | 3,00 |
| Sodium benzoate | 0,15 |
| Aq. dest. | 82,85 |
| | 100,00 |

Nach 1 Woche Behandlung mit der Pandanus-Nanoemulsion berichteten die Probanden eine starke Abnahme des Juckreizes (keine Beschwerden : 2 Probanden, milde Beschwerden 1 Proband), wohingegen die Anwendung mit Pflege-Lotio weniger Erleichterung im Vergleich zu der Anwendung mit Pandanus-Creme zeigte (mittelstarke Beschwerden 2 Probanden/ starke Beschwerden: 1 Proband).
Die Pandanus-Nanoemulsion-Creme scheint besser wirksam als die Pandanus-Microemulsion-Creme.

### Beispiel 14: Pandanusextrakt gegen Neurodermitis

Herstellung von Emulsionen unterschiedlicher Partikelgröße (Emulsion, Mikroemulsion, Nanoemulsion):

### Bestandteile der Emulsion-Formulierung :

| Nr. | Bestandteile | Anteil (Gew.-%) |
|---|---|---|
| 1 | Ethanolischer Pandanusextrakt (Beisp. 5) | 3,0 |
| 2 | Lipoid S 100 | 5,0 |
| 3 | Miglyol | 4,0 |
| 4 | Methylparaben | 0,4 |
| 5 | Propylparaben | 0,3 |
| 6 | Carrageen | 4,0 |
| 7 | Wasser | ad 100 |

Lipoid S 100 und Wasser werden zusammengegeben und unter Erwärmung bei ca. 50-60°C bis zur Homogenität gerührt. Danach wird Carrageen und anschließend die weiteren Stoffe zugegeben. Die Tröpfchengröße der Emulsion betrug > 50 um (gemessen mit PSS Accusizer)..

### Mikroemulsion-Formulierung :

Die Mikroemulsion in der Zusammensetzung wie in der Tab. 2 angegeben, wird anhand der oben beschriebenen Herstellungsmethode mittels Ultra-Turrax hergestellt.
Die Tröpfchengröße lag in einem Bereich von 1 bis 50 µm (gemessen mit PSS Accusizer).

### Nanoemulsion-Formulierung

Die Bestandteile wie in der Tab. 3 angegeben wurden unter Erwärmung mittels Ultra-Turrax voremulgiert. Die Mischung wurde anschließend mit einem Hochdruckhomogenisator bei 700 bar und 4 Zyklen homogenisiert (Avestin Emulsiflex). Die Tröpfchengröße wurde mittels PSS Nicomp bestimmt.
Die Nanoformulierung enthielt eine durchschnittliche Tröpfchengröße in einem Bereich von 100 bis 400 nm.

### Untersuchung an Neurodermitis-Probanden:

Eine Gruppe von drei Probanden, die an Neurodermitis leidet, wurde über einen Zeitraum von 1 Woche mit der konventionellen Emulsion behandelt.

Eine weitere Gruppe von drei Neurodermitis-Probanden erhielt über den gleichen Zeitraum die gleiche Menge der Mikroformulierung und eine dritte Gruppe von drei Neurodermitis-Probanden erhielt die Nanoformulierung.

Die befallene Stelle wurde zweimal täglich behandelt. Die Behandlungs- und Beobachtungszeit betrug eine Woche. Die Veränderung der Hauptsymptomatik der Erkrankung, nämlich Juckreiz, wurde vor und nach der Behandlung mit unterschiedlichen Formulierungen verglichen. Alle Probanden hatten vor Beginn der Behandlung ein starkes bis mittelstarkes Juckreiz-Leiden (Abstufung der Juckreiz-Symptomatik: starke/4 - mittelstarke/3 - milde/2 - keine Beschwerden/1).

Die Gruppe, welche die Makroformulierung erhielt, zeigt zwar eine Verbesserung der Neurodermitis wies jedoch insgesamt das schlechteste Resultat auf. Deutlich besser schnitten die Gruppen ab, welche die Mikroformulierung und die Nanoformulierung erhielten, wobei die Nanoformulierung noch eine leichte Verbesserung gegenüber der Mikroformulierung zu bewirken scheint.

Die Veränderungen der Hauptsymptomatik der Neurodermitis vor und nach zweimal-täglicher und einwöchiger Behandlung mit Pandanusextrakt in unterschiedlichen Formulierungen:

| Probanden | Makroformulierung | Mikroformulierung | Nanoformulierung |
|---|---|---|---|
| 1 | 4 → 3 | | |
| 2 | 4 → 3 | | |
| 3 | 3 → 3 | | |
| 4 | | 4 → 2 | |
| 5 | | 3 → 2 | |
| 6 | | 4 → 3 | |
| 7 | | | 3 → 2 |
| 8 | | | 4 → 1 |
| 9 | | | 4 → 2 |

Abstufung der Juckreiz-Symptomatik: starke (4) - mittelstarke (3) - milde (2) - keine Beschwerden (1)

### Beispiel 15: Effekt von Pandanus-Extrakt auf die LPS induzierte TNF-α Ausschüttung in humanen Monozyten

Für den Versuch wurden humane Monozyten von drei verschiedenen Spendern jeweils mit LPS (10ng/ml) in Abwesenheit und in Gegenwart von Pandanusextrakt in unterschiedlichen Konzentrationen stimuliert. Die Ergebnisse sind in der Figur 3 zusammengefasst und zeigen 100% TNF-α Ausschüttung nach Stimulation mit LPS ohne Pandanus-Extrakt und abnehmende TNF-α Ausschüttung in Abhängigkeit von der eingesetzten Pandanus-Extrakt Konzentration (n=3). Der IC-50 liegt bei < 10µg/ml.

### Beispiel 16: Effekt von Pandanus-Extrakt auf die LPS induzierte IL-6 Ausschüttung in humanen Monozyten

Für den Versuch wurden humane Monozyten von drei verschiedenen Spendern jeweils mit LPS (10ng/ml) in Abwesenheit und in Gegenwart von Pandanusextrakt in unterschiedlichen Konzentrationen stimuliert. Die Ergebnisse sind in der Figur 4 zusammengefasst und zeigen 100% IL-6 Ausschüttung nach Stimulation mit LPS ohne Pandanus-Extrakt und abnehmende IL-6 Ausschüttung in Abhängigkeit von der eingesetzten Pandanus-Extrakt Konzentration (n=3). Der IC-50 liegt bei < 10µg/ml.

### Beispiel 17: Effekt von Pandanus-Extrakt auf die LPS induzierte PGE-2 Ausschüttung in humanen Monozyten

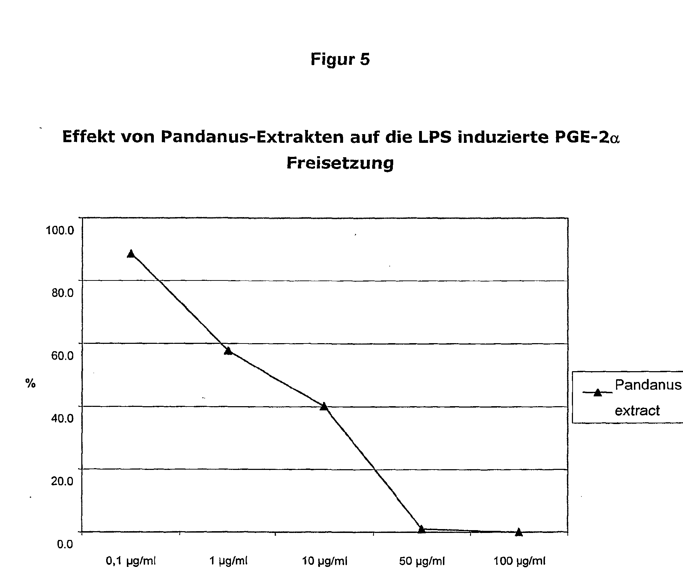

Für den Versuch wurden humane Monozyten von drei verschiedenen Spendern jeweils mit LPS (10ng/ml) in Abwesenheit und in Gegenwart von Pandanusextrakt in unterschiedlichen Konzentrationen stimuliert. Die Ergebnisse sind in der Figur 5 zusammengefasst und zeigen 100% PGE-2 Ausschüttung nach Stimulation mit LPS ohne Pandanus-Extrakt und abnehmende PGE-2 Ausschüttung in Abhängigkeit von der eingesetzten Pandanus-Extrakt Konzentration (n=3). Der IC-50 liegt bei < 50µg/ml.

### Beispiel 18: Erfahrungsbericht mit Pandanus-Extrakt-Nanoemulsionen zur Prophylaxe gegen Neurodermitis

Die Anwendung von Pandanus-Extrakt-Nanoemulsionen zur Prophylaxe gegen Neurodermitis wurde bei drei Probanden mit atopischem Ekzem und mit symmetrischen befallenen Stellen beobachtet. Nach einer einmonatigen Periode zur Aufnahme der Ausgangssymptome, wendeten die Probanden die Pandanus-Extrakt-Nanoemulsion zweimal täglich über einen Zeitraum von fünf Monaten an. Als primärer Endpunkt diente der Vergleich der von den Patienten berichteten Symptome mit den Ausgangssymptomen. Alle 3 Probanden hatten vor Beginn der Behandlung starke bis mittelstarke Juckreiz-Beschwerden (Abstufung der Juckreiz-Symptomatik: starke - mittelstarke - milde - keine Beschwerden). Nach zwei Wochen Behandlung mit der Pandanus-Extrakt-Nanoemulsion berichteten die Probanden von einer starken Abnahme des Juckreizes (keine Beschwerden : 3 Probanden). Die Probanden blieben über den gesamten Zeitraum der Studie von fünf Monaten weitestgehend Beschwerdefrei und berichteten nur von kurzen Phasen milden Juckreizes.

**Tab.4 Zusammensetzung der angewendeten Nanoemulsion-Creme zur Prophylaxe gegen Neurodermitis**

| | % |
|---|---|
| Dipalmitoyl-Phosphatidylcholin | 5,000 |
| Glycerol 85% | 1,500 |
| Dexpanthenol | 1,000 |
| Urea | 3,000 |
| Pandanusextrakt | 3,000 |
| Tocopherolacetat | 0,200 |
| Sodium benzoate | 0,150 |
| Aq. Dest. | 86,15 |
| | 100,00 |

### Beispiel 19: Erfahrungsbericht mit Pandanusextrakt-Nanoemulsionen ohne hautpflegende Zusatzstoffe

Die Anwendung von Pandanusextrakt-Nanoemulsion wurde bei fünf Probanden mit Psoriasis und mit symmetrischen befallenen Stellen beobachtet. Ein Teil der befallenen Stellen wurde mit einer Lotion freier Wahl und der andere Teil mit Pandanus-Nanoemulsion zweimal täglich behandelt (links-rechts-Vergleich). Als Lotion wählten die fünf Probanden eine Aloe-Vera-Creme mit 45% Aloe-Anteil folgender Zusammensetzung: 45% Aloe Vera, Menthol, Eucalyptusöl, WintergrünÖl, Jojoba-Öl, Aprikosenkernöl und Sesamöl. Die Behandlungs- und Beobachtungszeit war eine Woche. Die Veränderung der Hauptsymptomatik der Erkrankung, nämlich Juckreiz, wurde vor und nach der Behandlung und zwischen den Behandlungen verglichen. Alle 5 Probanden hatten vor Beginn der Behandlung ein starkes bis mittelstarkes Juckreiz-Leiden. (Abstufung der Juckreiz-Symptomatik: starke - mittelstarke - milde - keine Beschwerden):

**Tab.5 Zusammensetzung der Nanoemulsion nur mit Pandanusextrakt und Emulgatoren**

| | % |
|---|---|
| Dipalmitoyl-Phosphatidylcholin | 6,00 |
| Mygliol | 8,00 |
| Ethanolischer Pandanusextrakt (Beispiel 5) | 3,00 |
| Sodium benzoate | 0,15 |
| Aq. dest. | 82,85 |
| | 100,00 |

Nach 1 Woche Behandlung mit der Pandanus-Nanoemulsion berichteten die Probanden von einer starken Abnahme des Juckreizes (milde Beschwerden: 4 Probanden, keine Beschwerden: 1 Proband), wohingegen die Anwendung mit Aloe-Vera-Lotion weniger Erleichterung im Vergleich zu der Anwendung mit Pandanus-Creme zeigte (mittelstarke Beschwerden: 5 Probanden). Alle fünf Probanden berichteten zusätzlich, dass die mit Pandanusextrakt behandelte Seite, ein sichtbar verbessertes Hautbild zeigte.

### Beispiel 20: Erfahrungsbericht mit Pandanusextrakt-Nanoemulsionen zur Prophylaxe gegen Psoriasis

Die Anwendung von Pandanusextrakt-Nanoemulsionen zur Prophylaxe gegen Psoriasis wurde bei drei Probanden mit symmetrisch befallenen Stellen beobachtet. Nach einer einmonatigen Periode zur Aufnahme der Ausgangssymptome, wendeten die Probanden die Pandanusextrakt-Nanoemulsion zweimal täglich über einen Zeitraum von fünf Monaten an. Als primärer Endpunkt diente der Vergleich der von den Patienten berichteten Symptome mit den Ausgangssymptomen. Alle 3 Probanden hatten vor Beginn der Behandlung starke bis mittelstarke Juckreiz-Beschwerden (Abstufung der Juckreiz-Symptomatik: starke - mittelstarke - milde - keine Beschwerden). Nach zwei Wochen Behandlung mit der Pandanusextrakt-Nanoemulsion berichteten die Probanden von einer starken Abnahme des Juckreizes (keine Beschwerden: 2 Probanden, milde Beschwerden: 1 Proband). Die Probanden berichteten über den weiteren Zeitraum der Studie nur von kürzeren Phasen milden Juckreizes, der aber nicht mehr die Länge und Intensität der Ausgangssymptome erreichen konnte.

**Tab.6 Zusammensetzung der angewendeten Nanoemulsion-Creme zur Prophylaxe gegen Psoriasis**

| | % |
|---|---|
| Dipalmitoyl-Phosphatidylcholin | 5,000 |
| Glycerol 85% | 1,500 |
| Dexpanthenol | 1,000 |
| Urea | 3,000 |
| Pandanusextrakt | 3,000 |
| Tocopherolacetat | 0,200 |
| Sodium benzoate | 0,150 |
| Aq. Dest. | 86,15 |
| | 100,00 |

## Patentansprüche

1. Verwendung eines Extraktes gewonnen aus der Frucht der Spezies *Pandanus conoideus* zur Herstellung einer topischen Formulierung zur Behandlung und Prophylaxe von Erkrankungen der Haut, wobei die topische Formulierung eine Mikroemulsion oder eine Nanoemulsion mit einer durchschnittlichen Tröpfchengröße von 50nm bis zu 50µm enthält oder aus einer Mikroemulsion oder aus einer Nanoemulsion mit einer durchschnittlichen Tröpfchengröße von 50nm bis zu 50µm besteht, wobei es sich bei den Erkrankungen der Haut um ein atopisches Ekzem, Dermatitis, Psoriasis oder Akne handelt.

2. Verwendung nach Anspruch 1, wobei die topische Formulierung ein Trägersystem in Form einer Öl-in-Wasser-Emulsion oder einer Wasser-in-Öl-Emulsion enthält.

3. Verwendung nach Anspruch 1, wobei der Extrakt aus dem roten äußeren Fruchtfleisch und/oder aus dem weißen hellen inneren Fruchtfleisch und/oder aus den Kernen gewonnen wird.

4. Verwendung nach Anspruch 3, wobei der Extrakt aus dem roten äußeren Fruchtfleisch samt den Kernen oder nur aus dem roten Fruchtfleisch durch direkte Verpressung ohne jegliche chemische Zusätze gewonnen wird.

5. Verwendung nach Anspruch 3, wobei der Extrakt aus dem roten äußeren Fruchtfleisch samt den Kernen oder nur aus dem roten Fruchtfleisch durch Extraktion mit organischen Lösungsmitteln ausgewählt aus Methanol, Ethanol, Propanol, Isopropanol, Aceton, Ethylacetat oder Hexan gewonnen wird.

6. Verwendung nach Anspruch 3, wobei der Extrakt aus den Kernen ein hydrophiler Extrakt ist, der durch Abtrennung und Zerkleinerung der Kerne und Extraktion der zerkleinerten Kerne mit einem hydrophilen Lösungsmittel gewonnen wird.

7. Verwendung nach Anspruch 3, wobei der Extrakt aus dem weißen hellen inneren Fruchtfleisch ein hydrophiler Extrakt ist, der durch direkte Verpressung des abgetrennten weißen hellen inneren Fruchtfleisches oder durch Trocknung und Zerkleinerung des abgetrennten weißen hellen inneren Fruchtfleisches und anschließender Extraktion des getrockneten zerkleinerten weißen hellen inneren Fruchtfleisches mit einem hydrophilen Lösungsmittel gewonnen wird.

8. Verwendung nach Anspruch 3 , wobei der Extrakt aus dem roten äußeren Fruchtfleisch und/oder aus dem weißen hellen inneren Fruchtfleisch und/oder aus den Kernen mittels überkritischer Fluide gewonnen wird.

9. Verwendung nach einem der Ansprüche 1 - 8, wobei die topische Formulierung in Form einer Emulsionsformulierung, Salbe, oder Creme vorliegt.

## Claims

1. Utilisation of an extract obtained from the fruit of the species *Pandanus conoideus* for the production of a topical formulation for the treatment and prophylaxis of diseases of the skin, wherein the topical formulation contains a micro-emulsion or a nano-emulsion with an average droplet size of 50nm up to 50µm or consists of a micro-emulsion or of a nano-emulsion with an average droplet size of 50nm up to 50µm, wherein the disease of the skin is atopic eczema, dermatitis, psoriasis or acne.

2. Utilisation according to claim 1, wherein the topical formulation contains a carrier system in the form of an oil-in-water emulsion or a water-in-oil emulsion.

3. Utilisation according to claim 1, wherein the extract is obtained from the red outer pulp and/or from the white pale inner pulp and/or from the seeds.

4. Utilisation according to claim 3, wherein the extract is obtained from the red outer pulp together with the seeds or only from the red pulp by direct pressing without any chemical additives.

5. Utilisation according to claim 3, wherein the extract is obtained from the red outer pulp together with the seeds or only from the red pulp by extraction with organic solvents selected from methanol, ethanol, propanol, isopropanol, acetone, ethyl acetate or hexane.

6. Utilisation according to claim 3, wherein the extract from the seeds is a hydrophilic extract, which is obtained by separation and crushing of the seeds and extraction of the crushed seeds with a hydrophilic solvent.

7. Utilisation according to claim 3, wherein the extract from the white pale inner pulp is a hydrophilic extract, which is obtained by direct pressing of the separated white pale inner pulp or by drying and crushing of the separated white pale inner pulp and subsequent extraction of the dried crushed white pale inner pulp with a hydrophilic solvent.

8. Utilisation according to claim 3, wherein the extract is obtained from the red outer pulp and/or from the white pale inner pulp and/or from the seeds by use of supercritical fluids.

9. Utilisation according to one of the claims 1 - 8, wherein the topical formulation is present in the form of an emulsion formulation, ointment or cream.

## Revendications

1. Utilisation d'un extrait obtenu à partir du fruit de l'espèce *Pandanus conoideus* pour la production d'une formulation topique destinée au traitement et à la prophylaxie des maladies de la peau, dans laquelle la formulation topique contient une microémulsion ou une nanoémulsion ayant une taille moyenne des gouttelettes allant de 50nm jusqu'à 50µm ou consistant en une microémulsion ou une nanoémulsion ayant une taille moyenne des gouttelettes allant de 50nm jusqu'à 50µm, où la maladie de la peau est l'eczéma atopique, la dermatite, le psoriasis ou l'acné.

2. Utilisation selon la revendication 1, dans laquelle la formulation topique contient un système véhiculaire sous la forme d'une émulsion huile dans l'eau ou d'une émulsion eau dans l'huile.

3. Utilisation selon la revendication 1, dans laquelle l'extrait est obtenu à partir de la pulpe rouge extérieure et/ou de la pulpe blanche pâle intérieure et/ou des grains.

4. Utilisation selon la revendication 3, dans laquelle l'extrait est obtenu à partir de la pulpe rouge extérieure avec des graines ou seulement de la pulpe rouge par pressage direct sans aucun additif chimique.

5. Utilisation selon la revendication 3, dans laquelle l'extrait est obtenu à partir de la pulpe rouge extérieure avec des graines ou seulement de la pulpe rouge par extraction aux solvants organiques sélectionnés à partir du méthanol, de l'éthanol, du propanol, d'isopropanol, de l'acétone, de l'acétate d'éthyle ou de l'hexane.

6. Utilisation selon la revendication 3, dans laquelle l'extrait obtenu à partir des graines est un extrait hydrophilique, qui est obtenu par séparation et écrasage des graines et extraction des graines écrasées avec un solvant hydrophile.

7. Utilisation selon la revendication 3, dans laquelle l'extrait obtenu à partir de la pulpe blanche pâle intérieure est un extrait hydrophilique, qui est obtenu par pressage directe de la pulpe blanche pâle intérieure ou par séchage et écrasage de la pulpe blanche pâle intérieure séparée et extraction ultérieure de la sèche pulpe blanche pâle intérieure avec un solvant hydrophilique.

8. Utilisation selon la revendication 3, dans laquelle l'extrait est obtenu à partir de la pulpe rouge extérieure et/ou de la pulpe blanche pâle intérieure et/ou des graines en utilisant des fluides supercritiques.

9. Utilisation selon une des revendications 1 - 8, dans laquelle la formulation topique est présente sous la forme d'une formulation d'émulsion, d'un onguent ou d'une crème.
